# FASCICULE DE BREVET EUROPEEN

(11) **EP 2 417 111 B1**
(45) Date de publication et mention de la délivrance du brevet: **04.06.2014**
(21) Numéro de dépôt: 10723208.4
(22) Date de dépôt: 06.04.2010
(51) Int. Cl.: C07D 237/28, A61K 31/502, A61P 29/00

(54) **DERIVES DE 1-ALKYL-CINNOLIN-4(1H)-ONE SUBSTITUES,LEUR PREPARATION ET LEUR APPLICATION EN THERAPEUTIQUE**
SUBSTITUIERTE 1-ALKYLCINNOLIN-4-(1H)-ONDERIVATE, DEREN HERSTELLUNG UND DEREN THERAPEUTISCHE ANWENDUNG
SUBSTITUTED 1-ALKYLCINNOLIN-4(1H)-ONE DERIVATIVES, PREPARATION THEREOF AND THERAPEUTIC APPLICATION OF SAME

(30) Priorité: 07.04.2009 FR 0901696
(43) Date de publication de la demande: 15.02.2012
(73) Titulaire: SANOFI, 75008 Paris (FR)
(72) Inventeur: BARBAGALLO, Elodie, F-75013 Paris (FR); LEGEAY, Carole, F-75013 Paris (FR); RINALDI-CARMONA, Murielle, F-75013 Paris (FR); ROUX, Pascale, F-75013 Paris (FR); VERNHET, Claude, F-75013 Paris (FR)
(74) Mandataire: Tinel, Marie-Line
(86) Numéro de dépôt international: PCT/FR2010/050657
(87) Numéro de publication internationale: WO 2010/116084

(56) Documents cités:
- WO-A1-2009/053799
- STERN E ET AL: "Pharmacomodulations around the 4-oxo-1,4-dihydroquinoline-3-carboxam ides, a class of potent CB2-selective cannabinoid receptor ligands: consequences in receptor affinity and functionality" JOURNAL OF MEDICINAL CHEMISTRY, AMERICAN CHEMICAL SOCIETY, WASHINGTON, US, vol. 50, 1 janvier 2007 (2007-01-01), pages 5471-5484, XP002515712 ISSN: 0022-2623 [extrait le 2007-10-04] cité dans la demande

## Description

La présente invention a pour objet de nouveaux dérivés de 1-alkyl-cinnolin-4(1*H*)-one, ayant une affinité pour les récepteurs des cannabinoïdes de type 2 (CB₂), à leur préparation et à leur application en thérapeutique.

Le Δ⁹-THC est le principal constituant actif extrait de *Cannabis sativa* [Paton, Annual Review in Pharmacology (1975) 15 : 191-220].

De nombreux articles ont décrit non seulement des effets psychotropes des cannabinoïdes, mais aussi une influence de ces derniers sur la fonction immunitaire [Klein et al., Immunology Today (1998) 19 : 373-381], le contrôle de la douleur [Pertwee, Progress in Neurobiology (2001) 63 : 569-611], de la prise alimentaire [Cota et al. International Journal of Obesity (2003) 27 : 289-301] et de nombreuses autres fonctions biologiques [Nahas et al. Marihuana and medicine (1999) Humana Press : Totowa, New Jersey, USA].

Les effets des cannabinoïdes sont dus à une interaction avec des récepteurs spécifiques de haute affinité, couplés aux protéines G, présents au niveau central et périphérique [Howlett et al., Pharmacological Reviews (2002) 54 : 161-2002].

Les effets centraux des cannabinoïdes relèvent d'un premier type de récepteur (CB₁) présent principalement dans le cerveau mais aussi à la périphérie [Matsuda et al., Nature (1990) 346 : 561-564]. Par ailleurs, Munro et al. [Nature (1993) 365 : 61-65] ont cloné un second type de récepteur aux cannabinoïdes appelé CB₂ qui est présent à la périphérie et en particulier dans les cellules du système immunitaire, mais aussi dans le cerveau dans certaines conditions pathologiques.

Certains dérivés indoliques ont été cités dans l'art antérieur comme présentant une affinité pour les récepteurs CB₂ : on peut citer les demandes de brevets US 5 532 237, EP 833 818, US 4 581 354, WO 2002/42269, W02003/097597, WO 2006/069 196, WO 2007/057 571.

Des dérivés cinnoline ont été décrits par E. Stem et al. dans J. Med. Chem, 2007, 50, 5471-5484, en particulier le composé de formule :

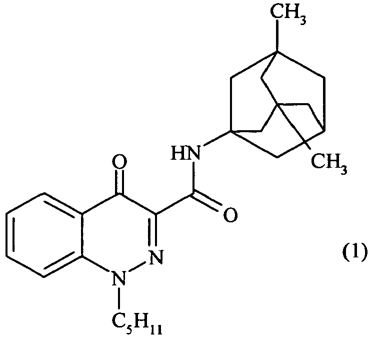

On a maintenant trouvé de nouveaux dérivés de 1-alkyl-cinnolin-4(1*H*)-one qui présentent un forte affinité et une grande sélectivité pour les récepteurs CB₂ des cannabinoïdes. Ces composés présentent un effet modulateur sur l'activité des récepteurs CB₂. Par effet modulateur on entend notament des effets agonistes, antagonistes et/ou agonistes inverses.

La présente invention a pour objet des composés répondant à la formule (I) : 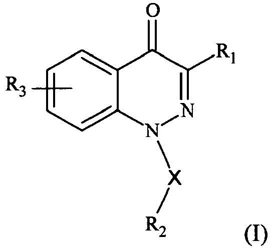 dans laquelle :
- X représente un radical bivalent (C₂-C₅)alkylène non substitué ou substitué une ou plusieurs fois par un groupe Alk ;
- R₁ représente :
   - un phényle substitué une ou plusieurs fois par des substituants choisis indépendamment parmi un atome d'halogène, un groupe Alk, un groupe OAlk;
   - un pyridyle substitué une ou plusieurs fois par des substituants choisis indépendamment parmi un atome d'halogène, un groupe Alk ;
- R₂ représente un atome d'hydrogène, un atome d'halogène, un groupe Alk, un groupe OAlk, un groupe -S-Alk, un groupe -SO-Alk, un groupe -SO₂-Alk, un groupe -CO-N(R₄)-Alk, un groupe -N(R₄)SO₂-Alk, un groupe - N(R₄)CO-Alk, un groupe -N(R₄)-SO₂-N(Alk)₂ ;
- R₃ représente un atome d'hydrogène, un atome d'halogène, un groupe Alk ou un groupe OAlk.
- R₄ représente un atome d'hydrogène ou un (C₁-C₄)alkyle ;
- Alk représente un (C₁-C₄)alkyle non substitué ou substitué une ou plusieurs fois par un atome de fluor ;
à l'état de base ou de sel d'addition à un acide.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables mais les sels d'autres acides utiles pour la purification ou l'isolement des composés de formule (I) font également partie de l'invention.

Lorsque les composés de formule (I) comprennent un atome de soufre, tous les isomères optiques ainsi que leur mélange en proportions quelconques sont objets de l'invention.

Par (C₂-C₅)alkylène, on entend un radical bivalent de deux à cinq atomes de carbone tel que le radical éthylène, triméthylène, tétraméthylène ou pentaméthylène.

Par (C₁-C₄)alkyle, on entend un radical alkyle linéaire ou ramifié de 1 à 4 atomes de carbone, tel que le radical méthyle, éthyle, propyle, isopropyle, n-butyle, isobutyle, *sec*-butyle, *tert*-butyle.

Par (C₁-C₄)alcoxy on entend un atome d'oxygène lié à un radical carboné linéaire ou ramifié de 1 à 4 atomes de carbone tel que le radical méthoxy, éthoxy, propoxy, isopropoxy, butoxy, isobutoxy, sec-butoxy, tert-butoxy.

Par atome d'halogène on entend un atome de brome, de chlore, de fluor ou d'iode.

Particulièrement, on préfère les composés de formule (I) dans laquelle :
- X représente un radical bivalent (C₂-C₅)alkylène non substitué ou substitué une ou plusieurs fois par un méthyle ;
- R₁ représente : 3-fluoro-2-trifluorométhylphényle, 2-fluoro-3-trifluorométhylphényle, 3-trifluorométhoxyphényle, 2-chloro-3-trifluorométhylphényle, 3-trifluorométhylphényle, 5-fluoro-2-méthoxyphényle, 4-fluoro-2-méthoxyphényle, 2,3-dichlorophényle, 2-(trifluorométhyl)pyridin-3-yle ;
- R₂ représente un atome d'hydrogène, un atome de fluor, de chlore, un radical trifluorométhyle, un groupe -OCH₃, un groupe -OCH₂CH₃, un groupe -S-CH₃, un groupe -S-CH₂-CH₃, un groupe -SO-CH₂-CH₃, un groupe -SO₂CH₃, un groupe - SO₂CH₂CH₃, un groupe -NHSO₂-CH₃, un groupe -NHSO₂-CF₃, un groupe - NHSO₂CHF₂, un groupe -N(CH₃)-SO₂CH₃, un groupe -NH-CO-CF₃, un groupe-N(CH₃)-CO-CF₃, un groupe -NH-SO₂-N(CH₃)₂, un groupe -CO-N(CH₃)₂, placés en position terminale de la chaîne alkyle ;
- R₃ représente un atome d'hydrogène, un atome de chlore, un groupe méthyle, un un groupe méthoxy ou un radical trifluorométhyle ;
à l'état de base ou de sel d'addition à un acide.

Parmi les composés de formule (I) objets de l'invention, on peut notamment citer les composés suivants :
- 3-[3-Fluoro-2-(trifluorométhyl)phényl]-8-méthoxy-1-pentylcinnolin-4(1*H*)-one ;
- 3-[2-Fluoro-3-(trifluorométhyl)phényl]-8-méthoxy-1-pentylcinnolin-4(1*H*)-one ;
- 3-[2-Fluoro-3-(trifluorométhyl)phényl]-1-pentylcinnolin-4(1*H*)-one ;
- 3-[2-Fluoro-3-(trifluorométhyl)phényl]-1-(5,5,5-trifluoropentyl)cinnolin-4(1*H*)-one ;
- 8-Méthoxy-1-pentyl-3-[3-(trifluorométhoxy)phényl]cinnolin-4(1*H*)-one ;
- 3-[2-Fluoro-3-(trifluorométhyl)phényl]-1-(4,4,4-trifluorobutyl)cinnolin-4(1*H*)-one ;
- 3-[2-Fluoro-3-(trifluorométhyl)phényl]-1-[3-(méthylthio)propyl]cinnolin-4(1*H*)-one;
- 1-(4-Fluorobutyl)-3-[2-fluoro-3-(trifluorométhyl)phényl]cinnolin-4(1*H*)-one ;
- 3-[2-Chloro-3-(trifluorométhyl)phényl]-8-méthoxy-1-pentylcinnolin-4(1*H*)-one ;
- 3-[3-(Trifluorométhyl)phényl]-1-(5,5,5-trifluoropentyl)cinnolin-4(1*H*)-one ;
- 3-[2-Méthoxy-5-(trifluorométhyl)phényl]-1-(5,5,5-trifluoropentyl)cinnolin-4(1*H*)-one ;
- 1-(3-Chloropropyl)-3-[3-(trifluorométhyl)phényl]cinnolin-4(1*H*)-one ;
- 1-Pentyl-3-[3-(trifluorométhyl)phényl]cinnolin-4(1*H*)-one ;
- 1-[3-(Méthylthio)propyl]-3-[3-(trifluorométhyl)phényl]cinnolin-4(1*H*)-one ;
- 3-(4-Fluoro-2-méthoxyphényl)-1-(4,4,4-trifluorobutyl)cinnolin-4(1*H*)-one ;
- 3-(5-Fluoro-2-méthoxyphényl)-1-(4,4,4-trifluorobutyl)cinnolin-4(1*H*)-one ;
- 7-Chloro-3-[2-chloro-3-(trifluorométhyl)phényl]-1-(5,5,5-trifluoropentyl)cinnolin-4(1H)-one ;
- N-(3-[3-[2-fluoro-3-(trifluorométhyl)phényl]-8-méthoxy-4-oxocinnolin-1(4*H*)-yl]propyl)méthanesulfonamide ;
- N-[3-[3-[2-chloro-3-(trifluorométhyl)phényl]-8-méthoxy-4-oxocinnolin-1(4*H*)-yl]propyl]méthanesulfonamide ;
- N-[3-[3-[3-fluoro-2-(trifluorométhyl)phényl]-8-méthoxy-4-oxocinnolin-1(4*H*)-yl]propyl]méthanesulfonamide ;
- N-[3-[3-(2,3-dichlorophényl-8-méthoxy-4-oxocinnolin-1(4*H*)-yl)propyl]méthanesulfonamide ;
- N-[3-[8-méthoxy-4-oxo-3-[2-(trifluorométhyl)pyridin-3-yl]cinnolin-1(4*H*)-yl]propyl]méthanesulfonamide ;
- N-[3-[3-(2,3-dichlorophényl)-4-oxo-7-(trifluorométhyl)cinnolin-1(4*H*)-yl]propyl]méthanesulfonamide ;
- N-{3-[3-[2-fluoro-3-(trifluorométhyl)-phényl]-4-oxo-7-trifluorométhyl-4*H*-cinnolin-1-yl]-propyl}-méthanesulfonamide ;
- N-{3-[3-[3-fluoro-2-(trifluorométhyl)-phényl]-4-oxo-7-trifluorométhyl-4*H*-cinnolin-1-yl]-propyl}-méthanesulfonamide ;
- N-{3-[3-[2-fluoro-3-(trifluorométhyl)-phényl]-4-oxo-4*H*-cinnolin-1-yl]-propyl}-méthanesulfonamide ;
- N-{3-[3-[2-fluoro-3-(trifluorométhyl)phényl]-7-méthoxy-4-oxo-4*H*-cinnolin-1-yl]-propyl}-méthanesulfonamide ;
- 1-[2-(Ethylthio)éthyl]-3-[2-fluoro-3-(trifluorométhyl)phényl]-8-méthoxycinnolin-4(1*H*)-one ;
- 1-[2-(Ethylsulfinyl)éthyl]-3-[2-fluoro-3-(trifluorométhyl)phényl]-8-méthoxycinnolin-4(1*H*)-one ;
- 1-[2-(Ethylsulfonyl)éthyl]-3-[2-fluoro-3-(trifluorométhyl)phényl]-8-méthoxycinnolin-4(1*H*)-one ;
- 3-(2,3-Dichlorophényl)-1-[2-(éthylthio)éthyl]-8-méthoxycinnolin-4(1*H*)-one ;
- 3-(2,3-Dichlorophényl)-1-[2-(éthylsulfinyl)éthyl]-8-méthoxycinnolin-4(1*H*)-one ;
- 3-(2,3-Dichlorophényl)-1-[2-(éthylsulfonyl)éthyl]-8-méthoxycinnolin-4(1*H*)-one ;
- N-(3-{8-méthoxy-4-oxo-3-[3-(trifluorométhoxy)phényl]cinnolin-1(4*H*)-yl}propyl)méthanesulfonamide ;
- 1,1,1-Trifluoro-N-(3-{3-[2-fluoro-3-(trifluorométhyl)phényl]-8-méthoxy-4-oxocinnolin-1(4*H*)-yl}propyl)méthanesulfonamide ;
- 2,2,2-Trifluoro-N-(3-{3-[2-fluoro-3-(trifluorométhyl)phényl]-8-méthoxy-4-oxocinnolin-1(4*H*)-yl}propyl)acétamide ;
- N-(3-{7-chloro-3-[2-fluoro-3-(trifluorométhyl)phényl]-4-oxocinnolin-1(4*H*)-yl}propyl)méthanesulfonamide ;
- N-{3-[3-(2,3-dichlorophényl)-8-méthoxy-4-oxocinnolin-1(4*H*)-yl]propyl}-N-méthylméthanesulfonamide ;
- 1-[2-(Ethylthio)éthyl]-3-[2-fluoro-3-(trifluorométhyl)phényl]-8-(trifluorométhoxy)cinnolin-4(1*H*)-one ;
- 7-Chloro-1 -[2-(éthylthio)éthyl]-3-[2-fluoro-3-(trifluorométhyl)phényl]cinnolin-4(1*H*)-one ;
- 7-Chloro-1-[2-(éthylsulfonyl)éthyl]-3-[2-fluoro-3-(trifluorométhyl)phényl]cinnolin-4(1*H*)-one ;
- 7-Chloro-3-(2,3-dichlorophényl)-1-[2-(éthylthio)éthyl]cinnolin-4(1*H*)-one ;
- 2,2,2-Trifluoro-N-(3-{3-[2-fluoro-3-(trifluorométhyl)phényl]-8-méthoxy-4-oxocinnolin-1(4*H*)-yl}propyl)-N-méthylacétamide ;
- N-(3-{7-chloro-3-[3-fluoro-2-(trifluorométhyl)phényl]-4-oxocinnolin-(4*H*)-yl}propyl)méthanesulfonamide ;
- 7-chloro-1-[2-(éthylthio)éthyl]-3-[3-(trifluorométhoxy)phényl]cinnolin-4(1*H*)-one ;
- 7-Chloro-3-(2,3-dichlorophényl)-1-[2-(éthylsulfinyl)éthyl]cinnolin-4(1*H*)-one ;
- 7-Chloro-3-(2,3-dichlorophényl)-1-[2-(éthylsulfonyl)éthyl]cinnolin-4(1*H*)-one ;
- 1-[2-(Ethylthio)éthyl]-8-méthoxy-3-[3-(trifluorométhoxy)phényl]cinnolin-4(1*H*)-one ;
- 1-[2-(éthylsulfonyl)éthyl]-8-méthoxy-3-[3-(trifluorométhoxy)phényl]cinnolin-4(1*H*)-one ;
- 3-(2,3-dichlorophényl)-1-(2-éthoxyéthyl)-8-méthoxycinnolin-4(1*H*)-one ;
- 1-(2-éthoxyéthyl)-3-[2-fluoro-3-(trifluorométhyl)phényl]-8-méthoxycinnolin-4(1*H*)-one ;
- N'-{3-[3-(2,3-dichlorophényl)-8-méthoxy-4-oxocinnolin-1(4*H*)-yl]propyl}-N,N-diméthylsulfamide ;
- 1,1-difluoro-N-(3-{3-[2-fluoro-3-(trifluorométhyl)phényl]-8-méthoxy-4-oxocinnolin-1(4*H*)-yl}propyl)méthanesulfonamide ;
- N-(2-{3-[2-fluoro-3-(trifluorométhyl)phényl]-8-méthoxy-4-oxocinnolin-1(4*H*)-yl}éthyl)méthanesulfonamide ;
- 3-{3-[2-fluoro-3-(trifluorométhyl)phényl]-8-méthoxy-4-oxocinnolin-1(4*H*)-yl}-N,N-diméthylpropanamide ;
- 3-[2-fluoro-3-(trifluorométhyl)phényl]-8-méthoxy-1-(3-méthoxy-3-méthylbutyl)cinnolin-4(1*H*)-one ;
- 3-(2,3-dichlorophényl)-8-méthoxy-1-(3-méthoxy-3-méthylbutyl)cinnolin-4(1*H*)-one ;
- 3-[3-fluoro-2-(trifluorométhyl)phényl]-8-méthoxy-1-(3-méthoxy-3-méthylbutyl)cinnolin-4(1*H*)-one ;
- 8-fluoro-3-[2-fluoro-3-(trifluorométhyl)phényl]-1-(3-méthoxy-3-méthylbutyl)cinnolin-4(1*H*)-one ;
à l'état de base ou de sel d'addition à un acide.

Dans ce qui suit, on entend par groupe protecteur GP un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans "Protective Group in Organic Synthesis", Green et al., 4th Edition (John Wiley & Sons, Inc., New York), 2007.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans "Advanced Organic Chemistry", M.B. Smith and J. March, 6th Edition, Wiley Interscience, 2007, p. 496-501.

Conformément à l'invention, on peut préparer les composés de formule (I) selon un procédé qui est caractérisé en ce que :
On fait réagir, en présence d'une base, un composé de formule : 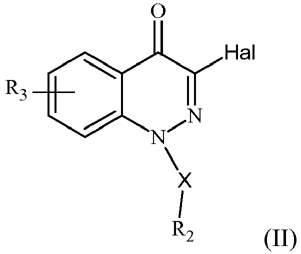 dans laquelle X, R₂ et R₃ sont tels que définis pour un composé de formule (I), et Hal représente un atome d'halogène avec un composé de formule :

   R₁-B(OH)₂ (III)
dans laquelle R₁ est tel que défini pour un composé de formule (I).

La réaction peut s'effectuer en présence d'un catalyseur au palladium tel que par exemple le tetrakis(triphénylphosphine)palladium, en présence d'une base telle que par exemple le carbonate de sodium, dans un solvant tel que par exemple le toluène, le méthanol, l'éthanol ou un mélange de ces solvants, à une température comprise entre la température ambiante et 100°C.

Eventuellement, on transforme le composé de formule (I) en l'un de ses sels d'addition avec des acides minéraux et/ou organiques.

Les composés de formule (I) ainsi obtenus peuvent être ultérieurement séparés du milieu réactionnel et purifiés selon les méthodes classiques, par exemple par cristallisation ou chromatographie.

Les composés de formule (I) ainsi obtenus sont isolés sous forme de base libre ou de sel, selon les techniques classiques.

Selon une variante de ce procédé, on peut également préparer un composé de formule (I) dans laquelle R₂ représente un groupe -S-Alk, par réaction d'un composé de formule : 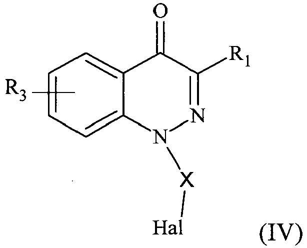 dans laquelle X, R₁ et R₃ sont tels que définis pour un composé de formule (I) et Hal représente un atome d'halogène, avec un dérivé d'alcanethiolate de sodium de formule :

NaS- Alk (V)

La réaction s'effectue dans un solvant tel que l'éthanol à une température comprise entre la température ambiante et la température de reflux du solvant.

Selon une autre variante des procédés ci-dessus on peut préparer un composé de formule (I) dans laquelle R₂ représente -SOAlk ou -SO₂Alk par réaction d'un composé de formule (I) dans laquelle R₂ représente -SAlk avec un agent oxydant. Comme agent oxydant, on peut utiliser par exemple l'eau oxygénée ou l'acide 3-chloroperbenzoïque, dans un solvant tel que le dichlorométhane et à une température comprise entre 0°C et la température ambiante.

Selon le nombre d'équivalents d'oxydant utilisé et selon la température de la réaction, on obtient un sulfoxyde (R₂ = -SOAlk) ou une sulfone (R₂ = -SO₂Alk). On peut également obtenir un mélange des deux composés que l'on sépare en utilisant des procédés connus de l'homme du métier, par exemple, par chromatographie préparative.

Les composés de formule (II) se préparent par réaction d'un composé de formule : 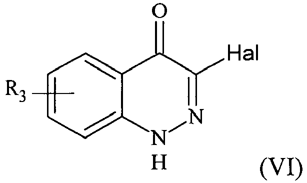 dans laquelle R₃ est tel que défini pour un composé de formule (I) et Hal représente un atome d'halogène avec un composé de formule:

Y-X-R₂ (VII)

dans laquelle X et R₂ sont tels que définis pour un composé de formule (I) et Y représente un atome d'halogène ou un hydroxy.

Lorsque Y = Hal, la réaction s'effectue en présence d'une base forte telle que par exemple l'hydrure de sodium, dans un solvant tel que par exemple le N,N-diméthylformamide à une température comprise entre 0° et la température de reflux du solvant.

Lorsque Y = OH, la réaction s'effectue en présence de diéthylazodicarboxylate et de triphénylphosphine dans un solvant tel que, par exemple, le tétrahydrofurane et à une température comprise entre 0°C et la température ambiante.

Selon une variante de ce procédé, on peut également préparer les composés de formule (II) dans laquelle R₂ = -N(R₄)-SO₂Alk avec R₄ = H, par réaction d'un composé de formule : 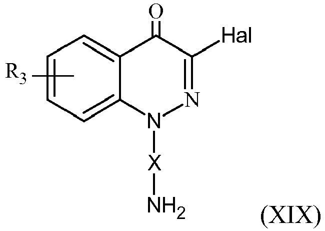 dans laquelle X et R₃ sont tels que définis pour un composé de formule (I) et Hal représente un atome d'halogène avec un composé de formule :

Hal-SO₂Alk (XX)

dans laquelle Alk est tel que défini pour un composé de formule (I) et Hal représente un atome d'halogène.

La réaction s'effectue en présence d'une base telle que par exemple la triéthylamine, dans un solvant tel que par exemple le dichlorométhane et à une température comprise entre 0° C et la température ambiante.

On peut préparer les composés de formule (II) dans laquelle R₂ = -N(R₄)SO₂Alk avec R₄ différent de H par réaction des composés de formule (II) dans laquelle R₂ = -NHSO₂Alk avec un agent alkylant, en présence d'une base. Comme agent alkylant on utilise, par exemple, un sulfate de dialkyle de formule SO₄(R₄)₂ ou un halogénure d'alkyle de formule R₄Hal, formules dans lesquelles R₄ = (C₁-C₄)alkyle et Hal représente un atome d'halogène, en présence d'une base forte telle que par exemple l'hydrure de sodium par exemple.

On peut préparer les composés de formule (II) dans laquelle R₂ = -N(R₄)SO₂-N(Alk)₂ selon les mêmes modes opératoires précédemment décrits pour R₂ = -N(R₄)SO₂Alk à partir d'un composé de formule (XIX) et d'un composé de formule Hal-SO₂-N(Alk)₂ (XXa).

Les composés de formule (III) sont commerciaux, connus ou préparés selon des méthodes connues de l'homme de l'art.

Les composés de formule (IV) se préparent par réaction d'un composé de formule : 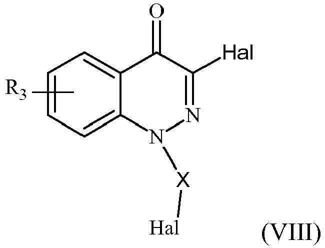 dans laquelle X et R₃ sont tels que définis pour un composé de formule (I) et Hal représente un atome d'halogène, avec un composé de formule (III).

La réaction s'effectue en présence d'un catalyseur au palladium tel que par exemple le tetrakis(triphénylphosphine)palladium, en présence d'une base telle que par exemple le carbonate de sodium, dans un solvant tel que par exemple le toluène, le méthanol, l'éthanol ou un mélange de ces solvants, à une température comprise entre la température ambiante et 100°C.

Les composés de formule (V) sont commerciaux, connus ou préparés selon des méthodes connues de l'homme de l'art.

Les composés de formule (VI) dans laquelle Hal réprésente un atome de brome, se préparent à partir d'un composé de formule : 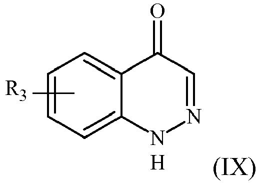 dans laquelle R₃ est tel que défini pour un composé de formule (I), par réaction avec du brome, en présence d'une base telle que par exemple l'éthylate de potassium, dans un solvant tel que par exemple l'acide acétique et à une température comprise entre la température ambiante et la température de reflux du solvant.

Les composés de formule (VI) dans laquelle Hal réprésente un atome d'iode se préparent par réaction d'un composé de formule (IX) avec de l'iode, en présence de phényl[bis(2,2,2-trifluoroacétoxy)]-λ³-iodane et d'une base telle que, par exemple, la pyridine, dans un solvant tel que, par exemple, le dichlorométhane et à température ambiante.

Les composés de formule (VII) sont commerciaux, connus ou préparés selon des méthodes connues de l'homme de l'art.

Les composés de formule (VIII) se préparent par réaction d'un composé de formule (VI) dans laquelle R₃ est tel que défini pour un composé de formule (I) avec un composé de formule:

Hal-X-Hal (X)

dans laquelle Hal représente un atome d'halogène et X est tel que défini pour un composé de formule (I). La réaction s'effectue en présence d'une base forte telle que par exemple l'hydrure de sodium, dans un solvant tel que par exemple le N,N-diméthylformamide à une température comprise entre 0° C et la température de reflux du solvant.

Les composés de formule (IX) se préparent par cyclisation d'un composé de formule : 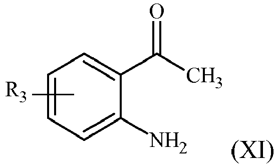 dans laquelle R₃ est tel que défini pour un composé de formule (I), en présence de nitrite de sodium, dans un solvant tel que par exemple l'acide chlorhydrique et à une température comprise entre 0°C et la température ambiante.

Les composés de formule (X) sont commerciaux, connus ou préparés selon des méthodes connues de l'homme de l'art.

Les composés de formule (XI) dans laquelle R₃ est tel que défini pour un composé de formule (I) se préparent selon le SCHEMA I ci-après.

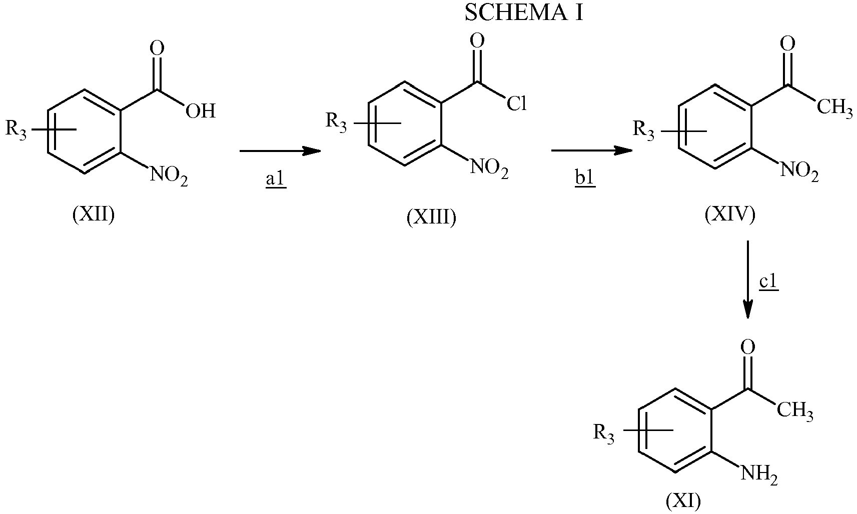

A l'étape a1 du SCHEMA I, la réaction d'un composé de formule (XII) avec du chlorure de sulfonyle s'effectue à une température comprise entre la température ambiante et 100°C.

A l'étape b1, le composé de formule (XIII) ainsi obtenu est mis en réaction avec du malonate de diéthyle, en présence de magnésium, dans un solvant tel que par exemple un éther (éther diéthylique par exemple) à une température comprise entre la température ambiante et la température de reflux du solvant.

Le composé (XIV) ainsi obtenu est réduit à l'étape c1 en présence de zinc, d'acide acétique dans un solvant tel que par exemple le tétrahydrofurane et à une température comprise entre 0°C et la température ambiante.

Le composé de formule (XII) est commercial, connu ou préparé selon des méthodes connues de l'homme de l'art.

Selon une variante de ce procédé, on peut également préparer un composé de formule (XI) dans laquelle R₃ est tel que défini pour un composé de formule (I) selon le SCHEMA II.

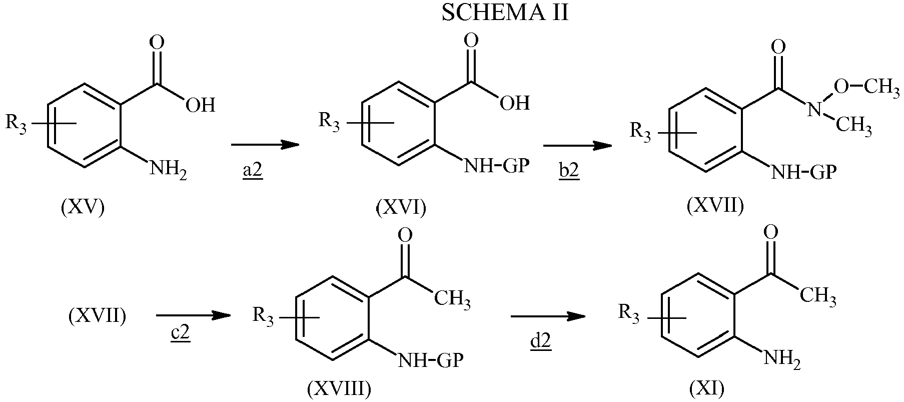

A l'étape a2 du SCHEMA II, on protège l'amine du composé formule (XV) selon les méthodes connues de l'homme du métier.

A l'étape b2, le composé de formule (XVI) ainsi obtenu est mis en réaction avec la N-méthoxyméthanamine, en présence d'un agent de couplage utilisé en chimie peptidique tel que par exemple le 1,3-dicyclohexylcarbodiimide (DCC) ou l'hexafluorophosphate de benzotriazol-1-yloxytris(diméthylamino) phosphonium (BOP) ou l'hexafluorophosphate de benzotriazol-1-yloxytris(pyrrolidino) phosphonium (PyBOP) ou le tétrafluoroborate de 2-(1*H*-benzotriazol-1-yl)-1,1,3,3-tétraméthyl uronium, en présence d'une base telle que par exemple la triéthylamine, la N,N-diisopropyléthyl amine ou la 4-diméthylaminopyridine, dans un solvant tel que par exemple le dichlorométhane, le dichloroéthane, le N-N-diméthylformamide ou le tétrahydrofurane à une température comprise entre -10°C et la température de reflux du solvant.

Le composé (XVII) ainsi obtenu est mis à réagir à l'étape c2 avec un composé organométallique tel que le bromure de méthylmagnésium dans un solvant tel qu'un éther (tétrahydrofurane, dioxane par exemple) à une température comprise entre -100°C et la température ambiante.

Le composé (XVIII) ainsi obtenu est déprotégé à l'étape d2.

Les composés (XIX) se préparent par réaction d'un composé de formule : 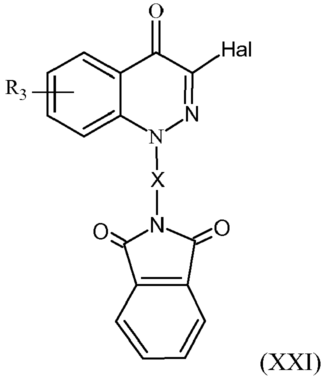 dans laquelle X et R₃ sont tels que définis pour un composé de formule (I) et Hal représente un atome d'halogène avec l'hydrazine monohydrate dans un solvant tel que par exemple le méthanol et à une température comprise entre la température ambiante et la température de reflux du solvant.

Les composés de formule (XX) et (XXa) sont commerciaux, connus ou préparés selon les méthodes connues de l'homme de l'art.

Les composés de formule (XXI) se préparent par réaction d'un composé de formule (VI) dans laquelle R₃ est tel que défini pour un composé de formule (I) avec un composé de formule : 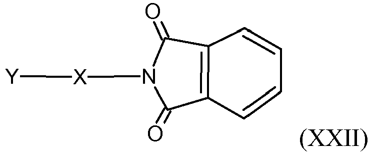 dans laquelle X est tel que défini pour un composé de formule (I) et Y représente un atome d'halogène ou un hydroxy.

A titre d'alternative au composé (XXII), on peut utiliser un composé de formule Hal-X-NHBOC avec le composé de formule (VI).

Lorsque Y = Hal, la réaction s'effectue en présence d'une base forte telle que par exemple l'hydrure de sodium, dans un solvant tel que par exemple le N,N-diméthylformamide à une température comprise entre 0°C et la température de reflux du solvant.

Lorsque Y = OH, la réaction s'effectue en présence de diéthylazodicarboxylate et de triphénylphosphine dans un solvant tel que, par exemple, le tétrahydrofurane et à une température comprise entre 0°C et la température ambiante.

Les composés de formule (XXII) sont commerciaux, connus ou préparés selon les méthodes connues de l'homme de l'art.

Les EXEMPLES suivants décrivent la préparation de certains composés conformes à l'invention. Ces exemples ne sont pas limitatifs et ne font qu'illustrer la présente invention. Les numéros des composés exemplifiés renvoient à ceux donnés dans le TABLEAU I ci-après, qui illustre les structures chimiques et les propriétés physiques de quelques composés selon l'invention.

Dans les Préparations et dans les Exemples on utilise les abréviations suivantes :
AcOEt : acétate d'éthyle,
AcONa : acétate de sodium,
BOC : *ter*-butyloxycarbonyle,
CLHP : chromatographie liquide haute performance,
DCM : dichlorométhane,
DIPEA : diisopropyléthylamine,
DMF : N,N-diméthylformamide,
DMSO : diméthylsulfoxyde,
éther : éther diéthylique,
éther iso : éther diisopropylique,
F : point de fusion,
HBr : acide bromhydrique,
MeOH : méthanol,
TA : température ambiante,
Tétrakis : tetrakis(triphénylphosphine)palladium,
TFA : acide trifluoroacétique,
THF : tétrahydrofurane.

Les spectres de résonance magnétique nucléaire du proton (RMN ¹H) sont enregistrés à 200 MHz dans le DMSO-d₆. Les déplacements chimiques δ sont exprimés en parties par million (ppm). Pour l'interprétation des spectres, on utilise les abréviations suivantes : s : singulet, d : doublet, t : triplet, qd : quadruplet, m : massif, mt : multiplet, se : singulet élargi, dd : doublet dédoublé.

Les mélanges de solvants sont quantifiés en rapport volumétriques.

Les composés selon l'invention sont analysés par couplage LC/UV/MS (Chromatographie Liquide/ détection UV/Spectrométrie de masse). On mesure le pic moléculaire (MH⁺) et le temps de rétention (tr) en minutes.

Les conditions utilisées sont les suivantes :
Conditions A
Colonne : Symmetry C₁₈ (2,1 x 50 mm) 3,5 µm ;
Eluant :
A : H₂O + 0,005 % TFA pH ≈ 3 ;
B : acétonitrile / 0,005 % TFA ;

Gradient :

| Temps (min.) | % A | % B |
|---|---|---|
| 0 | 100 | 0 |
| 10 | 10 | 90 |
| 15 | 10 | 90 |
| 16 | 0 | 100 |
| 20 | 0 | 100 |

Débit : 0,4 ml/minute ;
Détection UV : λ = 210-220 nm ;
Conditions B
On utilise une colonne XTerra MS C₁₈ (2,1 x 50 mm) 3,5 µm;
Eluant :
A : 10mM AcONH₄ pH ≈ 7 ;
B : acétonitrile ;

Gradient :

| Temps (mn) | % A | %B |
|---|---|---|
| 0 | 100 | 0 |
| 10 | 10 | 90 |
| 15 | 10 | 90 |
| 16 | 100 | 0 |
| 20 | 100 | 0 |

Débit : 0,4 ml/minute ;
Détection UV : λ = 220 nm ;
Conditions C
On utilise une colonne Acquity BEH C₁₈ (2,1 x 50 mm) 1,7 µm,
Eluant :
A : H₂O + 0,05 % TFA pH ≈ 3 ; acétonitrile (97/3)
B : acétonitrile / 0,035 % TFA ;

Gradient :

| Temps (mn) | % A | %B |
|---|---|---|
| 0 | 100 | 0 |
| 2,3 | 5 | 95 |
| 2,9 | 5 | 95 |
| 3 | 100 | 0 |

| Temps (mn) | % A | % B |
|---|---|---|
| 3,5 | 100 | 0 |

Débit : 1 ml/minute ;
Détection UV : λ = 220 nm ;

L'enregistrement des spectres de masse est effectué en mode electrospray (ESI) positif, afin d'observer les ions issus de la protonation des composés analysés (MH⁺) ou de la formation d'adduits avec d'autres cations tels que N⁺, K⁺, etc....

### PREPARATIONS

### 1. Préparations des composés de formule (XI).

### Préparation 1.1

### 1-(2-Amino-3-méthoxyphényl)éthanone.

### (XI) : R₃ = 3-OMe.

### A- Chlorure de 3-méthoxy-2-nitrobenzoyle.

On chauffe à 75°C pendant 3 heures un mélange de 10 g d'acide 3-méthoxy-2-nitrobenzoïque dans 60 ml de chlorure de thionyle. On concentre le mélange réactionnel sous vide et obtient 10,9 g du composé attendu que l'on utilise tel quel à l'étape suivante.

### B- 1-(3-méthoxy-2-nitrophényl)éthanone.

A un mélange de 3,2 g de magnésium dans 10 ml d'éther on ajoute, goutte à goutte et à TA, une solution de 20 ml de malonate de diéthyle dans 12 ml d'EtOH puis, goutte à goutte, une solution de 10,9 g du composé de l'étape précédente dans 40 ml d'éther et chauffe à reflux pendant 18 heures. On verse le mélange réactionnel dans 50 ml d'éther et filtre l'insoluble. On reprend l'insoluble par une solution saturée de NH₄Cl, extrait avec 100 ml de chloroforme, acidifie la phase aqueuse par ajout de 20 ml d'une solution d'HCl à 10 %, reextrait la phase aqueuse avec 100 ml de chloroforme, sèche les phases organiques jointes sur MgSO₄ et évapore les solvants sous vide. On reprend le résidu dans un mélange de 10 ml d'acide acétique, 1,5 ml d'H₂SO₄ et 7 ml d'eau puis chauffe à reflux pendant 5 heures. On concentre l'acide acétique sous vide, reprend le mélange réactionnel par 100 ml d'eau, alcalinise la phase aqueuse par ajout de NH₄OH, extrait avec 100 ml de chloroforme, lave la phase organique par une solution saturée de NaCl, sèche sur MgSO₄ et évapore le solvant sous vide. On chromatographie le résidu sur gel de silice en éluant par le gradient du mélange cyclohexane/AcOEt de (80/20; v/v) à (20/80; v/v). On obtient 3,3 g du composé attendu.

### C- 1-(2-Amino-3-méthoxyphényl)éthanone.

On refroidit à 0°C une solution de 3,3 g du composé de l'étape précédente dans 100 ml de THF, ajoute 13,27 g de zinc et 15,7 ml d'acide acétique puis laisse sous agitation en laissant remonter la température à TA et laisse 4 heures sous agitation à TA. On filtre le mélange réactionnel sur Célite^{®} et concentre le filtrat sous vide. On extrait le résidu au THF, lave la phase organique par 100 ml d'une solution de NaOH à 10%, par une solution saturée de NaCl, sèche sur MgSO₄ et évapore le solvant sous vide. On chromatographie le résidu sur gel de silice en éluant par le gradient du mélange cyclohexane/AcOEt de (80/20;v/v) à (60/40;v/v). On obtient 2 g du composé attendu.

### Préparation 1.2

### 1-(2-Amino-4-chlorophényl)éthanone.

### (XI) : R₃ = 4-Cl.

### A- Acide 2-[(tert-butoxycarbonyl)amino]-4-chlorobenzoique

A un mélange de 10 g d'acide 2-amino-4-chlorobenzoïque dans 45 ml de dioxane et 15 ml d'eau on ajoute 19,5 ml de triéthylamine puis, goutte à goutte, une solution de 14,73 g de di-tert-butyl dicarbonate dans 30 ml de dioxane et laisse 48 heures sous agitation à TA. On concentre le mélange réactionnel sous vide, reprend le résidu dans 100 ml d'un mélange eau/AcOEt, décante, lave la phase organique par une solution saturée de NaCl, sèche sur MgSO₄ et évapore le solvant sous vide. On chromatographie le résidu sur gel de silice en éluant par le gradient du mélange cyclohexane / AcOEt de (80/20;v/v) à (50/50;v/v). On obtient 5,35 g du composé attendu.

### B- [5-chloro-2-[méthoxy(méthyl)carbamoyl]phényl]carbamate de tert-butyle.

A une solution de 5,35 g du composé de l'étape précédente dans 200 ml de DCM on ajoute 8,78 ml de triéthylamine puis 2,11 g de N-méthoxyméthanamine et 11,27 g de PyBOP et laisse une nuit sous agitation à TA. On lave le mélange réactionnel à l'eau, par une solution de NaOH à 10%, par une solution saturée de NaCl, sèche sur MgS04 et évapore le solvant sous vide. On chromatographie le résidu sur gel de silice en éluant par le gradient du mélange cyclohexane/AcOEt de (80/20;v/v) à (60/40;v/v). On obtient 5,7 g du composé attendu.

### C- (2-Acétyl-5-chlorophényl)carbamate de tert butyle.

On refroidit à - 40°C une solution de 5,7 g du composé de l'étape précédente dans 445 ml de THF, ajoute, goutte à goutte, 38,8 ml de bromure de méthylmagnésium et laisse une nuit sous agitation à TA. On verse le mélange réactionnel sur une solution d'HCl à 10%, extrait à l'AcOEt, lave la phase organique par une solution saturée de NaCl, sèche sur MgSO₄ et évapore sous vide le solvant. On chromatographie le résidu sur gel de silice en éluant par le mélange cyclohexane/AcOEt (80/20;v/v) puis jusqu'à 100% d'AcOEt. On obtient 2,05 g du composé attendu.

### D- 1-(2-Amino-4-chlorophényl)éthanone.

A une solution de 0,9 g du composé de l'étape précédente dans 17 ml de DCM on ajoute, goutte à goutte, 2,55 ml d'acide trifluoroacétique et laisse une nuit sous agitation à TA. On lave le mélange réactionnel par une solution de NaOH à 10%, par une solution saturée de NaCl, sèche sur MgSO₄ et évapore le solvant. On obtient 0,6 g du composé attendu.

### 2. Préparations des composés de formule (IX).

### Préparation 2.1

### 8-methoxycinnolin-4(1H)-one.

### (IX) : R₃ = 8-OMe.

On refroidit à 0°C une solution de 2 g du composé de la préparation 1.2 dans 8 ml d'HCl concentré, ajoute, goutte à goutte, en maintenant la température du mélange réactionnel en dessous de 10°C, une solution de 1,25 g de NaNO₂ dans 2,7 ml d'eau, laisse 2 heures sous agitation à 0°C et une nuit à TA. On concentre le mélange réactionnel sous vide, ajoute 50 ml d'une solution d'AcONa, essore le précipité formé et le lave avec 20ml d'eau. On obtient 1,95 g du composé attendu.

### Préparation 2.2

### 7-chlorocinnolin-4(1H)-one.

### (IX) : R₃ = 7-Cl.

On refroidit à 0°C une solution de 1,8 g du composé de la préparation 1.2 dans 7 ml d'HCl concentré, ajoute, goutte à goutte, en maintenant la température du mélange réactionnel en dessous de 10°C, une solution de 1,1 g de NaNO₂ dans 2,39 ml d'eau, laisse 2 heures sous agitation à 0°C et une nuit à TA. On concentre le mélange réactionnel sous vide, ajoute 50 ml d'une solution d'AcONa, essore le précipité formé et le lave avec 20 ml d'eau. On obtient 1,6 g du composé attendu.

### 3. Préparations des composés de formule (VI).

### Préparation 3.1

### 3-bromo-8-méthoxycinnolin-4(1H)-one.

### (VI) : R₃ = 8-OMe ; Hal = Br.

A un mélange de 1,95 g du composé de la préparation 2.1 dans 20 ml d'AcOH, on ajoute 1,40 g de EtOK et chauffe à reflux. On ajoute ensuite, goutte à goutte, une solution de 0,68 ml de brome dans 2 ml d'AcOH et chauffe à reflux pendant 1 heure et 30 minutes. On concentre l'AcOH sous vide, reprend le résidu à l'eau, essore le précipité formé, le lave à l'eau et le sèche. On obtient 2,8 g du composé attendu.

### Préparation 3.2

### 3-bromo-7-chlorocinnolin-4(1H)-one.

### (VI) : R₃ = 7-Cl ; Hal = Br.

A un mélange de 1,8 g du composé de la préparation 2.2 dans 15 ml d'AcOH, on ajoute 1,26 g de EtOK et chauffe à reflux. On ajoute ensuite, goutte à goutte, une solution de 0,77 ml de brome dans 5 ml d'AcOH et chauffe à reflux pendant 3 heures. On concentre l'AcOH sous vide, reprend le résidu à l'eau, essore le précipité formé, le lave à l'eau et le sèche. On obtient 2,4 g du composé attendu.

### Préparation 3.3

### 3-bromocinnolin-4(1H)-one.

### (VI) : R₃ = H ; Hal = Br.

### Composé préparé selon le mode opératoire décrit dans la préparation 3.1.

### Préparation 3.4

### 3-iodo-8-méthoxycinnolin-4(1H)-one.

### (VI) : R₃ = 8-OMe ; Hal = I.

A un mélange de 9,5 g du composé de la préparation 2.1 et 13,91 g de phényl[bis(2,2,2-trifluoroacétoxy)]-λ³-iodane dans 270 ml de DCM on ajoute 8,2 g d'iode et 5,19 ml de pyridine. On laisse sous agitation une nuit à TA. On essore le précipité formé, le lave au n-pentane puis le sèche. On obtient 13,2 g du composé attendu.

### 4. Préparations des composés de formule (II).

### Préparation 4.1

### 3-bromo-8-méthoxy-1-pentylcinnolin-4(1H)-one.

### (II) : X = -(CH₂)₅-, R₂ = H, R₃ = 8-OMe ; Hal = Br.

On refroidit à 0-5°C une suspension de 0,33 g de NaH à 60% dans l'huile, dans 15 ml de DMF, ajoute goutte à goutte, une solution de 1,4 g du composé de la préparation 3.1 dans 15 ml de DMF puis une solution de 1,08 ml de 1-iodopentane dans 10 ml de DMF et chauffe à 75°C pendant 3 heures. On verse le mélange réactionnel dans l'eau, extrait au DCM, lave la phase organique par une solution saturée de NaCl, sèche sur MgSO₄, et évapore le solvant sous vide. On chromatographie le résidu sur gel de silice en éluant par le gradient du mélange cyclohexane/AcOEt de (80/20;v/v) à (50/50;v/v). On obtient 0,65 g du composé attendu.

### Préparation 4.2

### 3-bromo-7-chloro-1-(5,5,5-trifluoropentyl)cinnolin-4(1H)-one.

### (II) : X = -(CH₂)₄-, R₂ = CF₃, R₃ = 7-Cl ; Hal = Br.

On refroidit de 0 à 5°C une suspension de 0,55 g de NaH à 60% dans l'huile, dans 20 ml de DMF, ajoute goutte à goutte, une solution de 2,4 g du composé de la préparation 3.2 dans 30 ml de DMF puis une solution de 2,8 mg de 5-bromo-1,1,1-trifluoropentane dans 10 ml de DMF et chauffe à 75°C pendant 3 heures. On verse le mélange réactionnel dans l'eau, extrait au DCM, lave la phase organique par une solution saturée de NaCl, sèche sur MgSO₄, et évapore le solvant sous vide. On chromatographie le résidu sur gel de silice en éluant par le gradient du mélange cyclohexane/AcOEt de (80/20;v/v) à (40/60;v/v). On obtient 1,4 g du composé attendu.

### Préparation 4.3

### N-[3-(3-bromo-8-méthoxy-4-oxocinnolin-1(4H)-yl)propyl]méthanesulfonamide.

### (II) : X = -(CH₂)₃-, , R₂ = -NHSO₂Me, R₃ = 8-OMe ; Hal = Br.

### A- 2-[3-(3-Bromo-8-méthoxy-4-oxocinnolin-1(4H)-yl)propyl]-1H-isoindole-1,3(2H)-dione (XXI).

On refroidit à 0-5° C une suspension de 0,77 g de NaH à 60% dans l'huile, dans 15 ml de DMF, ajoute goutte à goutte, une solution de 3,3 g du composé de la préparation 3.1 dans 50 ml de DMF puis une solution de 5,2 g de 2-(3-bromopropyl)-1H-isoindole-1,3(2H)-dione dans 20 ml de DMF et chauffe à 75 °C pendant 6 heures et laisse revenir à TA pendant une nuit.

On verse le mélange réactionnel dans l'eau, extrait au DCM, lave la phase organique par une solution saturée de NaCl, sèche sur MgS04 et évapore le solvant sous vide. On chromatographie le résidu sur gel de silice en éluant par le gradient du mélange cyclohexane/AcOEt de (90/10 ; v/v) à (40/60 ; v/v). On obtient 5 g du composé attendu.

### B- 1-(3-Aminopropyl)-3-bromo-8-méthoxycinnolin-4(1H)-one (XIX).

A une solution de 3 g du composé de l'étape précédente dans 20 ml de MeOH on ajoute 0,66 ml d'hydrazine monohydrate et chauffe à reflux pendant 4 heures. On concentre le mélange réactionnel sous vide, reprend le résidu par une solution de NaOH à 10%, extrait au DCM, lave la phase organique par une solution saturée de NaCl, sèche sur MgSO₄ et évapore le solvant sous vide. On obtient 1,87 g du composé attendu.

### C- N-[3-(3-bromo-8-méthoxy-4-oxocinnolin-1(4H)-yl)propyl] méthanesulfonamide.

On laisse une nuit sous agitation à TA un mélange de 1,87 g du composé de l'étape précédente, 1,84 ml de triéthylamine et 0,51 ml de chlorure de méthanesulfonyle dans 75 ml de DCM. On lave le mélange réactionnel par une solution d'HCl à 10 %, par une solution de NaOH à 10 %, essore le précipité formé et le sèche. On obtient 2 g du composé attendu.

### Préparation 4.4

### 1,1,1-Trifluoro-N-[3-(3-iodo-8-méthoxy-4-oxocinnolin-1(4H)-yl)propyl]méthanesulfonamide.

### (II) : X = -(CH₂)₃- ; R₂ = -NHSO₂CF₃ ; R₃ = 8-OMe ; Hal = I

On refroidit à 5°C une suspension de 0,3 g de NaH à 60% dans l'huile dans 5 ml de DMF, ajoute, goutte à goutte, une solution de 0,75 g du composé de la préparation 3.4 dans 10 ml de DMF puis une solution de 2 g du composé de la préparation 7 dans 10 ml de DMF et laisse sous agitation en laissant remonter la température à TA. On chauffe à 100°C pendant 48 heures. On verse le mélange réactionnel dans l'eau, extrait au DCM, lave la phase organique par une solution saturée de NaCl, sèche sur MgSO₄, et évapore le solvant sous vide. On chromatographie le résidu sur gel de silice en éluant par le gradient du mélange cyclohexane/AcOEt de (80/20 ; v/v) à (50/50 ; v/v). On obtient le composé attendu.

### Préparation 4.5

### 8-Fluoro-3-iodo-1-(3-méthoxy-3-méthylbutyl)cinnolin-4(1H)-one.

### (II) : X = -CH₂CH₂C(CH₃)₂- ; R₂ = -OCH₃ ; R₃ = 8-F ; Hal = I.

On refroidit à 5°C un mélange de 0,72 g de 8-fluoro-3-iodocinnolin-4(1H)-one et 0,48 ml de 3-méthoxy-3-méthylbutan-1-ol dans 20 ml de THF, ajoute 0,97 g de triphénylphosphine puis lentement 0,65 g de diéthylazodicarboxylate et laisse une nuit sous agitation à TA. On concentre le mélange réactionnel sous vide et chromatographie le résidu sur gel de silice en éluant par le mélange cyclohexane/AcOEt (75/25 ; v/v) puis à l'AcOEt. On obtient le composé attendu.

### Préparation 4.6

### N-[2-(3-iodo-8-méthoxy-4-oxocinnolin-1(4H)yl)éthyl]méthanesulfonamide.

### (II) : X = -(CH₂)₂- ; R₂ = -NHSO₂Me ; R₃ = 8-OMe ; Hal = I.

### A- 2-[2-(3-Iodo-8-méthoxy-4-oxocinnolin-1(4H)éthyl)-1H-isoindole-1,3(2H)-dione (XXI).

On refroidit à 5°C un mélange de 2 g du composé de la préparation 3.4 et 2,5 g de 2-(2-hydroxyéthyl)-1*H*-isoindole-1,3(2*H*)-dione dans 50 ml de THF, ajoute 3,47 g de triphénylphosphine puis lentement 2,3 g de diéthylazodicarboxylate. Après 45 minutes sous agitation à TA, on concentre le mélange réactionnel sous vide, reprend le résidu au DCM et essore le précipité formé. On obtient 3,15 g du composé attendu.

### B- Chlorhydrate de 1-(2-aminoéthyl)-3-iodo-8-méthoxycinnolin-4(1H)-one (XIX).

A une solution de 1,5 g du composé de l'étape précédente dans 12 ml de MeOH on ajoute 0,31 ml d'hydrazine monohydrate et chauffe à 65°C pendant 3 heures. On concentre le mélange réactionnel sous vide, reprend le résidu par une solution de NaOH à 10%, extrait au DCM, lave la phase organique par une solution saturée de NaCl, sèche sur MgSO₄ et évapore le solvant sous vide. On dissout le produit ainsi obtenu dans un mélange DCM/MeOH, ajoute de l'éther chlorhydrique 2N et essore le précipité formé. On obtient 0,88 g du composé attendu.

### C- N-[2-(3-iodo-8-méthoxy-4-oxocinnolin-1(4H)yl)éthyl]méthanesulfonamide.

On laisse 48 heures sous agitation à TA un mélange de 0,4 g du composé de l'étape précédente, 0,58 ml de triéthylamine et 0,09 ml de chlorure de méthanesulfonyle dans 20 ml de DCM. On dilue le mélange réactionnel par ajout d'un mélange DCM/eau et essore le précipité formé. On obtient 0,21 g du composé attendu.

### 5. Préparations des composés de formule (VIII).

### 3-bromo-1-(3-chloropropyl)cinnolin-4(1H)-one.

### (VIII) : X = -CH₂)₃-,, R₃ = H, Hal = Cl

On refroidit à 5°C une suspension de 1,06 g de NaH à 60% dans l'huile dans 80 ml de DMF, ajoute, goutte à goutte, une solution de 4 g du composé de la préparation 3.3 dans 50 ml de DMF puis une solution de 2,64 ml de 1-bromo-3-chloropropane dans 50 ml de DMF et laisse une nuit sous agitation en laissant remonter la température à TA. On verse le mélange réactionnel dans l'eau, extrait au DCM, lave la phase organique par une solution saturée de NaCl, sèche sur MgSO₄, et évapore le solvant sous vide. On obtient 4,9 g du composé attendu.

### 6. Préparations des composés de formule (IV).

### 1-(3-chloropropyl)-3-[2-fluoro-(3-trifluorométhyl)phenyl]cinnolin-4(1H)-one.

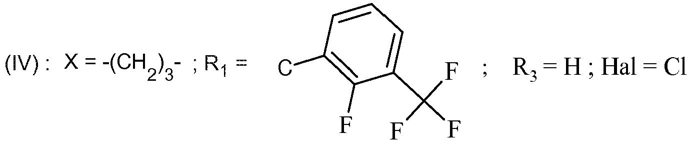

A un mélange de 1,4 g du composé de la préparation 5 dans 90 ml d'un mélange de toluène/MeOH (70/30 ; v/v), on ajoute ensuite 1,16 g d'acide (2-fluoro-3-trifluorométhylphényl) boronique puis 7,66 ml d'une solution 2M de Na₂CO₃. On fait barboter de l'azote dans le mélange réactionnel pendant 30 minutes, ajoute ensuite 1,07 g de Tétrakis et chauffe à reflux pendant 1 heure 30 minutes. On filtre le mélange réactionnel sur Célite^{®}, puis concentre le filtrat sous vide. On extrait le résidu au DCM, lave la phase organique par une solution de 10% de NaOH, par une solution saturée de NaCl, sèche sur MgSO₄ et évapore le solvant sous vide. On chromatographie sur gel de silice en éluant par le mélange cyclohexane/AcOEt (70/30 ; v/v) jusqu'à 100% d'AcOEt. On obtient 3,3 g du composé attendu.

### 7. Préparations des composés de formule (VII).

### N-(3-bromopropyl)-1,1,1-trifluorométhanesulfonamide.

### (VII) : X = -(CH₂)₃- ; Y = Br ; R₂ = -NHSO₂CF₃.

A une solution de 2 g de 3-bromopropan-1-amine dans 180 ml de DCM, on ajoute, sous atmosphère d'azote, 5,1 ml de triéthylamine puis 1,06 ml de chlorure de trifluorométhanesulfonyle et laisse une nuit sous agitation à TA. On lave le mélange réactionnel par une solution tampon pH = 2, par une solution saturée de NaCl, sèche la phase organique sur MgSO₄ et évapore le solvant sous vide. On obtient 0,48 g du composé attendu sous forme d'huile.

### EXEMPLE 1 : Composé N° 2

### 3-[2-fluoro-3-(trifluorométhyl)phényl]-8-méthoxy-1-pentylcinnolin-4(1H)-one.

A une solution de 0,15 g du composé de la Préparation 4.1 dans 10 ml d'un mélange de toluène/MeOH (70/30 ; v/v), on ajoute ensuite 0,11 g d'acide [2-fluoro-3-(trifluorométhyl)phényl]boronique puis 0,76 ml d'une solution 2M de Na₂CO₃. On fait barboter de l'azote dans le mélange réactionnel pendant 20 minutes, ajoute 0,11 g de Tétrakis et chauffe à 80°C pendant 18 heures. On filtre le mélange réactionnel sur Célite^{®} et lave à l'AcOEt. On lave le filtrat par 30 ml de NaOH à 10%, par une solution saturée de NaCl, sèche sur MgSO₄ et évapore les solvants sous vide. On chromatographie le résidu sur gel de silice en éluant par le gradient du mélange cyclohexane/AcOEt de (90/10;v/v) à (70/30;v/v). On obtient 0,11 g du composé attendu, F = 82-84 °C.

RMN ¹H : DMSO-*d*₆ (250 MHz) : δ (ppm) : 0.84 : t : 3 H ; 1.22 - 1.42 : m : 4 H; 1.69 - 1.95 : m : 2H ; 4.03 : s : 3H ; 4.61 - 4.86 : m : 2H ; 7.42 - 7.61 : m : 3H; 7.74 -8.00 : m : 3 H.

### EXEMPLE 2 : Composé N° 17

### 7-chloro-3-[2-chloro-3-(trifluoromethyl)phenyl]-1-(5,5,5-trifluoropentyl)-4a,8a-dihydrocinnolin-4(1H)-one.

A une solution de 0,25 g du composé de la Préparation 4.2 dans 13 ml d'un mélange de toluène/MeOH (70/30 ; v/v), on ajoute 0,16 g d'acide [2-chloro-3-(trifluorométhyl)phényl]boronique puis 1,08 ml d'une solution 2M de Na₂CO₃. On fait barboter de l'azote dans le mélange réactionnel pendant 20 minutes, ajoute 0,15 g de Tétrakis et chauffe à 80°C pendant 18 heures. On filtre le mélange réactionnel sur Célite^{®} et lave à l'AcOEt. On lave le filtrat par 30 ml de NaOH à 10%, par une solution saturée de NaCl, sèche sur MgSO₄ et évapore les solvants sous vide. On chromatographie le résidu sur gel de silice en éluant par le gradient du mélange cyclohexane/AcOEt de (90/10;v/v) à (70/30;v/v). On obtient 0,19 g du composé attendu, F = 137-138 °C.

RMN ¹H : DMSO-*d*₆ (250 MHz) : δ (ppm) : 1.46 - 1.71 : m : 2 H ; 1.78 - 2.01 : m:2H;2.16-2.42:m:2H;4.57:t:2H;7.55-7.64:m, 1 H; 7.69 : t : 1 H ; 7.77 - 7.85 : m : 1 H ; 7.94 - 8.04 : m ; 1 H ; 8.17 - 8.26 : m : 2 H.

### EXEMPLE 3 : Composé N°7

### 3-(2-fluoro-3-(trifluorométhyl)phényl)-1-[3-(methylthio)propyl]cinnolin-4(1H)-one.

On chauffe à reflux pendant 18 heures un mélange de 0,33 g du composé de la Préparation 6, de 0,12 g de méthanethiolate de sodium, dans 5 ml d'éthanol puis laisse 24 heures sous agitation. On concentre le mélange réactionnel sous vide extrait le résidu au DCM, lave la phase organique par une solution saturée de NaCl, sèche sur MgSO₄, filtre, et évapore le solvant sous vide. On chromatographie le résidu sur gel de silice en éluant par le gradient du mélange cyclohexane/AcOEt de (80/20;v/v) à (50/50;v/v). On obtient 0,21 g du composé attendu, F = 80-83 °C.

RMN ¹H : DMSO-d₆ (250 MHz,) : δ (ppm) : 1.99 : s : 3 H ; 2.02 - 2.16 : m : 2 H ; 2.55 : :t:2H;4.52-4.67:m:2H;7.43-7.60:m:2H;7.77-8.01 :m:4H;8.13 -8.25:m:1H.

### EXEMPLE 4 : Composé N°18

*N*-(3-[3-[2-fluoro-3-(trifluorométhyl)phényl]-8-méthoxy-4-oxocinnolin-1(4*H*)-yl]propyl)méthanesulfonamide.

A une solution de 0,3 g du composé de la Préparation 4.3 dans 15 ml d'un mélange toluène/MeOH (70/30 ; v/v), on ajoute 0,19 g d'acide [2-fluoro-3-(trifluorométhyl)phényl]boronique puis 1,27 ml d'une solution 2M de Na₂CO₃. On fait barboter de l'azote dans le mélange réactionnel pendant 20 minutes, ajoute 0,18 g de Tétrakis et chauffe à 75°C pendant une nuit. On filtre le mélange réactionnel sur Célite®, lave le filtrat par une solution de NaOH à 10%, par une solution saturée de NaCl, sèche la phase organique sur MgSO₄ et évapore les solvants sous vide. On chromatographie le résidu sur gel de silice en éluant par le mélange H₂O/MeOH (50/50 ; v/v). On obtient 0,085 g du composé attendu, F = 62-64°C.

RMN ¹H : DMSO-*d*₆ (250 MHz,) : δ (ppm) : 1.98-2.10 : m : 2 H ; 2.88 : s : 3 H ; 3.00-3.10 : m : 2 H ; 4.05 : s : 3 H ; 4.75-4.85 : m : 2 H ; 7.08 : t : 1H ; 7.50-7.60 : m : 3 H; 7.80-8.00 : m : 3H.

### EXEMPLE 5 : Composé N°33

### 3-(2,3-Dichlorophényl)-1-[2-(éthylsulfinyl)éthyl]-8-méthoxycinnolin-4(1H)-one.On refroidit à 5°C une solution de 0,104 g du composé N° 32 dans 3 ml d'acide acétique, ajoute 0,03 ml d'une solution à 30% d'H₂O₂ dans l'eau et laisse 2 heures sous agitation en laissant remonter la température à TA. On concentre le mélange réactionnel sous vide, extrait le résidu au DCM, lave la phase organique par une solution de NaOH à 10%, par une solution saturée de NaCl, sèche sur MgSO₄ et évapore le solvant sous vide. On obtient 0,047 g du composé attendu après cristallisation dans le mélange DCM/éther, F = 128-130°C.

### EXEMPLE 6 : Composé N°34

### 3-(2,3-Dichlorophényl)-1-[2-(éthylsulfonyl)éthyl]-8-méthoxycinnolin-4(1H)-one.

On refroidit à 5°C une solution de 0,245 g d'acide 3-chloroperbenzoïque dans 5 ml de DCM, ajoute goutte à goutte, une solution de 0,16 g du composé N° 32 dans 10 ml de DCM et laisse 1 heure 30 minutes sous agitation en laissant remonter la température à TA. On lave le mélange réactionnel par une solution de NaOH à 10%, par une solution saturée de NaCl, sèche la phase organique sur MgSO₄ et évapore le solvant sous vide. On obtient 0,15 g du composé, F = 127-129°C.

### EXEMPLE 7 : Composé N°60

### 8-Fluoro-3-[2-fluoro-3-(trifluorométhyl)phényl]-1-(3-méthoxy-3-méthylbutyl)cinnolin-4(1H)-one.

A une solution de 0,4 g du composé de la Préparation 4.5 dans 25 ml d'un mélange toluène/MeOH (70/30 ; v/v), on ajoute 0,25 g d'acide [2-fluoro-3-(trifluorométhyl)phényl]boronique puis 1,5 ml d'une solution 2M de Na₂CO₃. On fait barboter de l'azote dans le mélange réactionnel pendant 10 minutes, ajoute 0,24 g de Tétrakis et chauffe à 65°C pendant 1 heure et 30 minutes, puis agite une nuit à TA. On filtre le mélange réactionnel sur Célite® et concentre le filtrat sous vide. On chromatographie le résidu sur gel de silice en éluant par le gradient du mélange cyclohexane/AcOEt de (90/10 ; v/v) puis (65/35 ; v/v) jusqu'à (20/80 ; v/v). On obtient 0,24 g du composé attendu.

### EXEMPLE 8 : Composé N°55

### N-(2-{3-[2-fluoro-3-(trifluorométhyl)phényl]-8-méthoxy-4-oxocinnolin-1(4H)-yl} éthyl)méthanesulfonamide.

A une solution de 0,21 g du composé de la Préparation 4.6 dans 12 ml d'un mélange toluène/MeOH (75/25 ; v/v), on ajoute 0,124 g d'acide [2-fluoro-3-(trifluorométhyl)phényl]boronique puis 0,74 ml d'une solution 2M de Na₂CO₃. On fait barboter de l'azote dans le mélange réactionnel pendant 10 minutes, ajoute 0,115 g de Tétrakis et chauffe à 65°C pendant 1 heure et 30 minutes. On filtre le mélange réactionnel sur Célite® et concentre le filtrat sous vide. On chromatographie le résidu sur gel de silice en éluant par le mélange cyclohexane/AcOEt de (85/15 ; v/v) puis à l'AcOEt. On reprend le produit obtenu dans un mélange éther/DCM et essore le précipité formé. On obtient 0,045 g du composé attendu.

Le tableau qui suit illustre les structures chimiques et les propriétés physiques de quelques exemples de composés selon l'invention, obtenus en suivant les modes opératoires décrits dans les Exemples ci-dessus.

**TABLEAU I**

| | | | | |
|---|---|---|---|---|
| 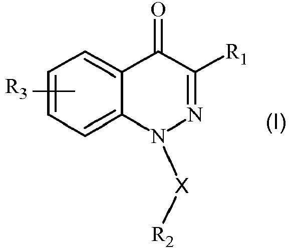 | | | | |

| **Composé No.** | **R₁** | **-X-R₂** | **R₃** | **Sel** |
|---|---|---|---|---|
| | | | | **F°C ;** |
| | | | | **MH⁺ ; tr (min.)** |
| | | | | **Conditions** |
| **1** | 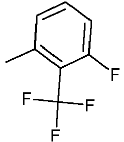 | -(CH₂)₄-CH₃ | 8-OCH₃ | - |
| | | | | - |
| | | | | 409 ; 1,99 |
| | | | | C |
| **2** | 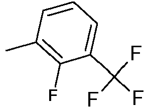 | -(CH₂)₄-CH₃ | 8-OCH₃ | - |
| | | | | 82-84°C |
| | | | | 409 ; 11,07 |
| | | | | B |
| **3** | 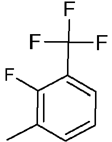 | -(CH₂)₄-CH₃ | H | - |
| | | | | 78-80°C |
| | | | | 379 ; 11,01 |
| | | | | A |
| **4** | 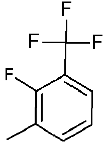 | -(CH₂)₄-CF₃ | H | - |
| | | | | 118-120°C |
| | | | | 433 ; 10,47 |
| | | | | A |
| **5** | 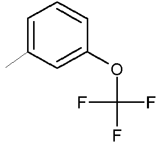 | -(CH₂)₄-CH₃ | 8-OCH₃ | - |
| | | | | 50-52°C |
| | | | | 407 ; 2,24 |
| | | | | C |
| **6** | 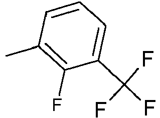 | -(CH₂)₃-CF₃ | H | - |
| | | | | 96-99°C |
| | | | | 419 ; 10,22 |
| | | | | A |
| **7** | 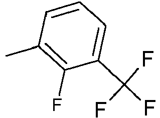 | -(CH₂)₃-S-CH₃ | H | - |
| | | | | 80-83°C |
| | | | | 397 ; 10,08 |
| | | | | A |
| **8** | 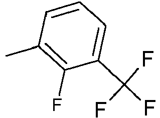 | -(CH₂)₄-F | H | - |
| | | | | 91-93°C |
| | | | | 383 ; 9,74 |
| | | | | A |
| **9** | 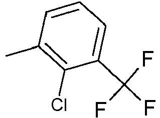 | -(CH₂)₄-CH₃ | H | - |
| | | | | 90-91°C |
| | | | | 425 ; 11,57 |
| | | | | A |
| **10** | 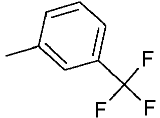 | -(CH₂)₄-CF₃ | H | - |
| | | | | 80-81°C |
| | | | | 415 ; 10,94 |
| | | | | A |
| **11** | 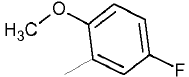 | -(CH₂)₄-CF₃ | H | - |
| | | | | 97-98°C |
| | | | | 395 ; 9,5 |
| | | | | A |
| **12** | 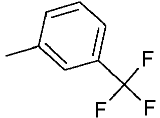 | -(CH₂)₃-Cl | H | - |
| | | | | - |
| | | | | 367 ; 10,45 |
| | | | | A |
| **13** | 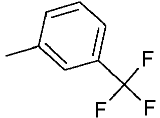 | -(CH₂)₄-CH₃ | H | - |
| | | | | 53-55°C |
| | | | | 361 : 18,48 |
| | | | | A |
| **14** | 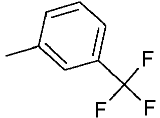 | -(CH₂)₃-S-CH₃ | H | - |
| | | | | - |
| | | | | 379 ; 10,64 |
| | | | | A |
| **15** | 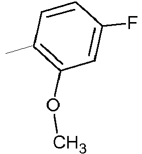 | -(CH₂)₃-CF₃ | H | - |
| | | | | - |
| | | | | 381 ; 9,25 |
| | | | | A |
| **16** | 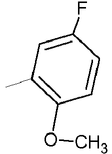 | -(CH₂)₃-CF₃ | H | - |
| | | | | 118-121°C |
| | | | | 381 ; 9,24 |
| | | | | A |
| **17** | 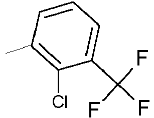 | -(CH₂)₄-CF₃ | 7-Cl | - |
| | | | | 137-138°C |
| | | | | 483 ; 1,96 |
| | | | | C |
| **18** | 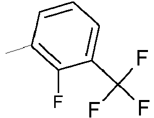 | -(CH₂)₃-NHSO₂Me | 8-OCH₃ | - |
| | | | | 62-64°C |
| | | | | 474 ; 1,41 |
| | | | | C |
| **19** | 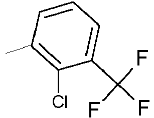 | -(CH₂)₃-NHSO₂Me | 8-OCH₃ | - |
| | | | | 95-98°C |
| | | | | 490 ; 1,42 |
| | | | | C |
| **20** | 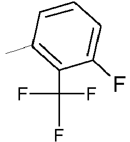 | -(CH₂)₃-NHSO₂Me | 8-OCH₃ | - |
| | | | | 66-68°C |
| | | | | 474 ; 1,31 |
| | | | | C |
| **21** | 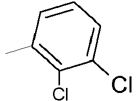 | -(CH₂)₃-NHSO₂Me | 8-OCH₃ | - |
| | | | | 66-68°C |
| | | | | 456 ; 1,35 |
| | | | | C |
| **22** | 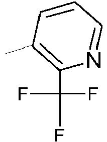 | -(CH₂)₃-NHSO₂Me | 8-OCH₃ | HCl |
| | | | | 187-189°C |
| | | | | 457 ; 1,23 |
| | | | | C |
| **23** | 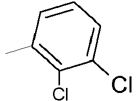 | -(CH₂)₃-NHSO₂Me | 7-CF₃ | |
| | | | | 80-83° C |
| | | | | 494 ; 1,52 |
| | | | | C |
| **24** | 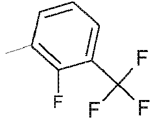 | -(CH₂)₃-SO₂Me | 8-OCH₃ | - |
| | | | | 138-139 |
| | | | | 459 ; 1,41 |
| | | | | C |
| **25** | 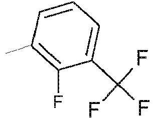 | -(CH₂)₃-NHSO₂Me | 7-CF₃ | - |
| | | | | 128-130 |
| | | | | 512 ; 1,57 |
| | | | | C |
| **26** | 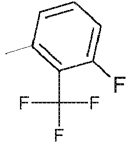 | -(CH₂)₃-NHSO₂Me | 7-CF₃ | - |
| | | | | - |
| | | | | 512 ; 1,49 |
| | | | | C |
| **27** | 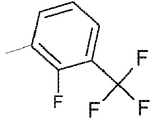 | -(CH₂)₃-NHSO₂Me | H | - |
| | | | | 149-152 |
| | | | | 444 ; 1,32 |
| | | | | C |
| **28** | 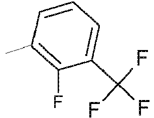 | -(CH₂)₃-NHSO₂Me | 7-OCH₃ | - |
| | | | | 54-56 |
| **29** | 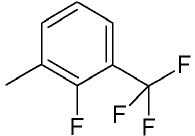 | (CH₂)₂-S-CH₂-CH₃ | 8-OCH₃ | - |
| | | | | 73-75 |
| | | | | 427 ; 1,92 |
| | | | | C |
| **30** | 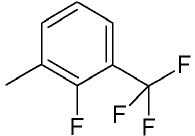 | (CH₂)₂-SO-CH₂-CH₃ | 8-OCH₃ | - |
| | | | | 99-101 |
| | | | | 443 ; 1,33 |
| | | | | C |
| **31** | 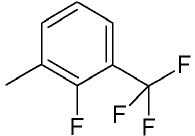 | (CH₂)₂-SO₂-CH₂-CH₃ | 8-OCH₃ | - |
| | | | | 138-139 |
| | | | | 459 ; 1,5 |
| | | | | C |
| **32** | 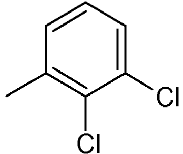 | (CH₂)₂-S-CH₂-CH₃ | 8-OCH₃ | - |
| | | | | - |
| | | | | 409 ; 1,89 |
| | | | | C |
| **33** | 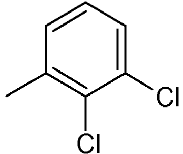 | (CH₂)₂-SO-CH₂-CH₃ | 8-OCH₃ | - |
| | | | | 128-130 |
| | | | | 425 ; 1,26 |
| | | | | C |
| **34** | 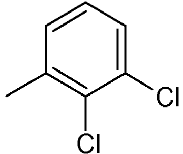 | (CH₂)₂-SO₂-CH₂-CH₃ | 8-OCH₃ | - |
| | | | | 127-129 |
| | | | | 441 ; 1,44 |
| | | | | C |
| **35** | 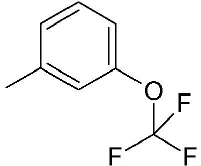 | (CH₂)₃-NH-SO₂-CH₃ | 8-OCH₃ | - |
| | | | | 98-101 |
| | | | | 472 ; 1,58 |
| | | | | C |
| **36** | 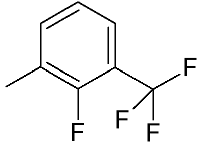 | (CH₂)₃-NH-SO₂-CF₃ | 8-OCH₃ | - |
| | | | | 127-130 |
| | | | | 528 ; 1,76 |
| | | | | C |
| **37** | 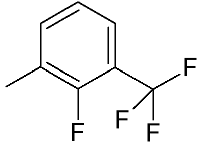 | (CH₂)₃-NH-CO-CF₃ | 8-OCH₃ | - |
| | | | | 51-53 |
| | | | | 492 ; 1,65 |
| | | | | C |
| **38** | 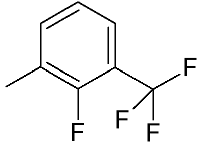 | (CH₂)₃-NH-SO₂-CH₃ | 7-Cl | - |
| | | | | 155-157 |
| | | | | 478 ; 1,5 |
| | | | | C |
| **39** | 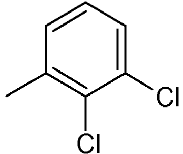 | (CH₂)₃-N(CH₃)-SO₂-CH₃ | 8-OCH₃ | - |
| | | | | 179-181 |
| | | | | 470 ; 1,51 |
| | | | | C |
| **40** | 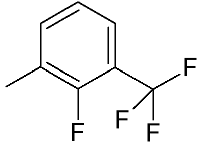 | (CH₂)₂-S-CH₂-CH₃ | 8-OCF₃ | - |
| | | | | - |
| | | | | 481;2,1 |
| | | | | C |
| **41** | 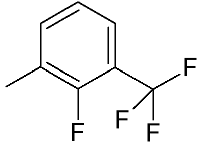 | (CH₂)₂-S-CH₂-CH₃ | 7-Cl | - |
| | | | | 117-119 |
| | | | | 431 ; 2 |
| | | | | C |
| **42** | 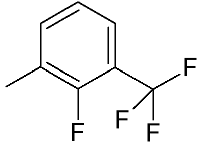 | (CH₂)₂-SO₂-CH₂-CH₃ | 7-Cl | - |
| | | | | 183-184 |
| | | | | 469 ; 1,56 |
| | | | | C |
| **43** | 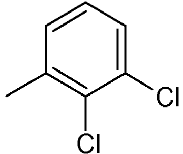 | (CH₂)₂-S-CH₂-CH₃ | 7-Cl | - |
| | | | | 75-77 |
| | | | | 413 ; 1,94 |
| | | | | C |
| **44** | 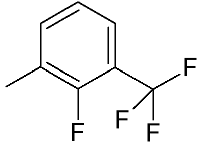 | (CH₂)₃-N(CH₃)-CO-CF₃ | 8-OCH₃ | - |
| | | | | - |
| | | | | 506 ; 1,77 |
| | | | | C |
| **45** | 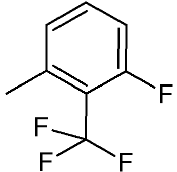 | (CH₂)₃-NH-SO₂-CH₃ | 7-Cl | - |
| | | | | 177-179 |
| | | | | 478 ; 1,41 |
| | | | | C |
| **46** | 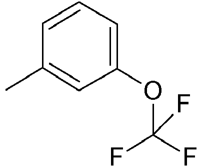 | (CH₂)₂-S-CH₂-CH₃ | 7-Cl | - |
| | | | | 66-68 |
| | | | | 429 ; 2,11 |
| | | | | C |
| **47** | 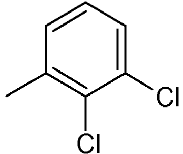 | (CH₂)₂-SO-CH₂-CH₃ | 7-Cl | - |
| | | | | 75-77 |
| | | | | 429 ; 1,34 |
| | | | | C |
| **48** | 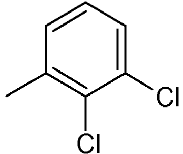 | (CH₂)₂-SO₂-CH₂-CH₃ | 7-Cl | - |
| | | | | 76-79 |
| | | | | 445 ; 1,51 |
| | | | | C |
| **49** | 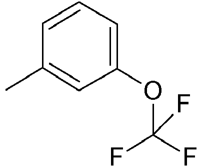 | (CH₂)₂-S-CH₂-CH₃ | 8-OCH₃ | - |
| | | | | 86-89 |
| | | | | 425 ; 2,07 |
| | | | | C |
| **50** | 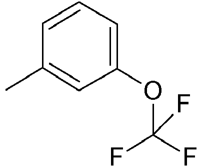 | (CH₂)₂-SO₂-CH₂-CH₃ | 8-OCH₃ | - |
| | | | | 130-132 |
| | | | | 457 ; 1,64 |
| | | | | C |
| **51** | 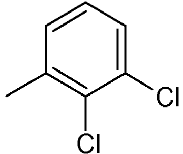 | (CH₂)₂-O-CH₂-CH₃ | 8-OCH₃ | - |
| | | | | 134-135 |
| | | | | 393 ; 1,7 |
| | | | | C |
| **52** | 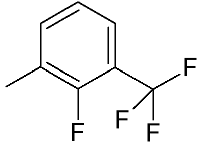 | (CH₂)₂-O-CH₂-CH₃ | 8-OCH₃ | - |
| | | | | 90-93 |
| | | | | 411 ; 1,75 |
| | | | | C |
| **53** | 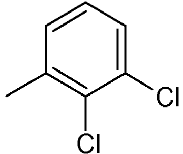 | (CH₂)₃-NH-SO₂-N-(CH₃)₂ | 8-OCH₃ | - |
| | | | | 62-65 |
| | | | | 485 ; 1,51 |
| | | | | C |
| **54** | 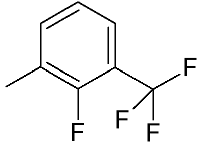 | (CH₂)₃-NH-SO₂-CHF₂ | 8-OCH₃ | - |
| | | | | - |
| | | | | 510 ; 1,66 |
| | | | | C |
| **55** | 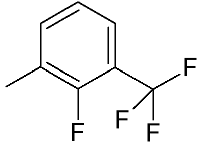 | (CH₂)₂-NH-SO₂-CH₃ | 8-OCH₃ | 145-146 |
| | | | | 460 ; 1,73 |
| | | | | C |
| **56** | 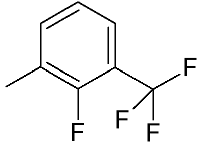 | (CH₂)₂-CO-N(CH₃)₂ | 8-OCH₃ | - |
| | | | | - |
| | | | | 438 ; 1,73 |
| | | | | C |
| **57** | 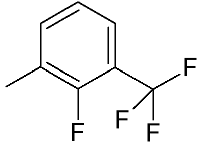 | (CH₂)₂-C(CH₃)₂-OCH₃ | 8-OCH₃ | - |
| | | | | - |
| | | | | 439 ; 2,17 |
| | | | | C |
| **58** | 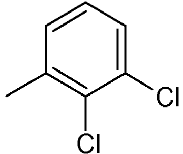 | (CH₂)₂-C(CH₃)₂-OCH₃ | 8-OCH₃ | - |
| | | | | - |
| | | | | 421 ; 2,13 |
| | | | | C |
| **59** | 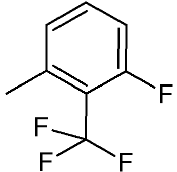 | (CH₂)₂-C(CH₃)₂-OCH₃ | 8-OCH₃ | - |
| | | | | - |
| | | | | 439 ; 2,07 |
| | | | | C |
| **60** | 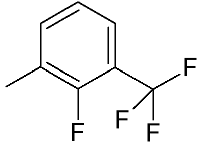 | (CH₂)₂-C(CH₃)₂-OCH₃ | 8-F | - |
| | | | | - |
| | | | | 427 ; 2,2 |
| | | | | C |

Les composés selon l'invention ont montré une très bonne affinité in vitro (CI50 < 500 nM) pour les récepteurs CB₂ humain et de rongeur. Les essais de liaison par affinité (binding) ont été réalisés selon les conditions expérimentales décrites par M. Rinaldi-Carmona dans J. Pharmacol. Exp. Therap. 1998, 287, 644-650, avec des membranes issues soit de tissus de rongeur, soit de lignée cellulaires recombinantes dans lesquelles les récepteurs CB₂ humains ont été exprimés (Munro et al., Nature 1993, 365, 61-65). L'affinité des composés est exprimée sous forme de CI50 (concentration causant une inhibition de 50% de la liaison spécifique du ligand tritié utilisé in vitro).

Les composés selon l'invention ont montré un effet modulateur sur les récepteurs CB₂. Notamment, les composés selon l'invention présentent des propriétés de nature agoniste, agoniste inverse et/ou antagoniste.

La nature agoniste des composés selon l'invention a été démontrée dans les modèles d'inhibition de l'adénylate-cyclase (stimulée par de la forskoline) comme décrit dans M. Rinaldi-Carmona et al., J. Pharmacol. Exp. Ther. 1996, 278, 871-878 et 1998, 284, 644-650 et M Bouaboula et al., J. Biol Chem., 1997, 272, 22330-22339.

La nature antagoniste des composés selon l'invention a été démontrée dans les modèles de reversion de l'inhibition de l'adénylate-cyclase (stimulée par de la forskoline) induite par un agoniste des récepteurs CB₂ comme décrit dans M. Rinaldi-Carmona et al., J. Pharmacol. Exp. Ther. 1996, 278, 871-878 et 1998, 284, 644-650.

La nature agoniste inverse des composés selon l'invention a été démontrée dans les modèles d'activation de l'adénylate-cyclase (stimulée par de la forskoline) comme décrit dans M Portier et al., J. Pharmacol. Exp. Ther. 1999, 288, 582-589.

Les composés selon l'invention possèdent également une bonne affinité *in vivo* pour les récepteurs CB₂ présents au niveau de la rate de souris lorsqu'ils sont administrés par voie orale. Les essais ont été réalisés selon les conditions expérimentales décrites par Rinaldi-Carmona et al., J. Pharmacol. Exp. Ther., 1998, 284, 644-650. L'affinité des composés est exprimée sous forme de DE50 (dose causant une inhibition de 50% de la liaison spécifique du ligand tritié utilisé ex vivo).

Les composés de la présente invention sont notamment des principes actifs compatibles avec leur utilisation en tant que médicaments et/ou compositions pharmaceutiques.

Selon un de ses aspects, la présente invention concerne l'utilisation d'un composé de formule (I) ou d'un de ses sels, pharmaceutiquement acceptable pour la préparation de médicaments destinés à prévenir ou à traiter toute pathologie humaine et/ou à usage vétérinaire. Ainsi les composés selon l'invention peuvent être utilisés chez l'homme ou chez l'animal (notamment chez les mammifères incluant de façon non limitative les chiens, les chats, les chevaux, les bovins, les moutons) pour la prévention ou le traitement de maladies impliquant les récepteurs CB₂.

On peut par exemple citer les maladies ou affections ci-après :
- Les désordres du système immunitaire : notamment les maladies auto-immunes incluant de façon non exhaustive : le psoriasis, le lupus érythémateux, les maladies du tissu conjonctif ou connectivites, le syndrome de Sjögrer's, la spondylarthrite ankylosante, la polyarthrite rhumatoïde, l'arthrite réactionnelle, la spondylarthrite indifférenciée, la maladie de Charcot, la maladie de Behcet's, les anémies auto-immunes hémolytiques, la sclérose en plaques, la sclérose latérale amyotrophique, les amyloses, le rejet de greffe, et les maladies affectant la lignée plasmocytaire ;
- Les maladies allergiques : notament l'hypersensibilité retardée ou immédiate, l'asthme, la rhinite allergique, la dermatite de contact et conjonctivite allergique ;
- Les maladies infectieuses parasitaire, virale ou bactérienne incluant notamment le SIDA et les méningites ;
- L'amylose et les maladies affectant les lignées du système lympho-hématopoïétique ;
- Les maladies chronique du foie d'origine alcoolique, la cirrhose, virale et toxique ainsi que les stéatohépatites d'origine non alcoolique et le cancer primaire du foie ;
- Les maladies inflammatoires : notamment les maladies articulaires: l'arthrite, l'arthrite rhumatoïde, l'ostéoarthrite, la spondylite, la goutte, la vascularite, la maladie de Crohn, la maladie du colon irritable syndrome de colon irritable (en anglais IBD : inflammatory bowel disease et IBS : irritable bowel syndrome) et la colite ulcérative pancréatite aiguë.
- Les maladies osseuses et de l'ostéoporose.
- La douleur : notamment les douleurs chroniques de type inflammatoire, les douleurs neuropathiques et les douleurs aiguës périphériques.
- Les affections oculaires : notamment l'hypertension oculaire et le glaucome.
- Les affections pulmonaires : maladies des voies respiratoires, asthme, bronchite chronique, obstruction chronique des voies respiratoires (en anglais COPD : chronic obstructive pulmonary disease), emphysème.
- Les maladies du système nerveux central et les maladies neuro dégénératives :notamment le syndrome de Tourette, la maladie de Parkinson, la maladie d'Alzheimer, la démence sénile, la chorée, la chorée de Huntington, l'épilepsie, les psychoses, la dépression et les lésions de la moelle épinière.

Le composé de formule (I) selon l'invention peut être utilisé comme médicament pour le traitement ou la prévention de la migraine, du stress, des maladies d'origine psychosomatique, des crises d'attaques de panique, de l'épilepsie, des troubles du mouvement, les vertiges, les vomissements, les nausées en particulier ceux consécutifs à une chimiothérapie.
- La migraine, le stress, les maladies d'origine psychosomatique, les crises de panique (attaque de panique ou crise d'angoisse aigüe), d'épilepsie, des troubles du mouvement, les vertiges, les vomissements, les nausées en particulier ceux consécutifs à une chimiothérapie.
- Les maladies cardiovasculaires en particulier hypertension, artériosclérose, crise cardiaque, ischémie cardiaque.
- L'ischémie rénale.
- Les cancers : notamment les tumeurs bénignes de la peau, papillomes et tumeurs cancéreuses, les tumeurs de la prostate, les tumeurs cérébrales (exemples : glioblastomes, médullo-épithéliomes, médulloblastomes, neuroblastomes, tumeurs d'origine embryonnaires, astrocytomes, astroblastomes, épendymomes, oligodendrogliomes, tumeur du plexus, neuro-épithéliomes, tumeur de l'épiphyse, épendymoblastomes, neuroectodermique, méningiomes malins, sarcomatoses, mélanomes malins, schwannomes).
- Les troubles gastro-intestinaux, les troubles diarrhéiques, les ulcères, les troubles vésicaux et urinaires, les néphrites, les troubles d'origine endocrinienne, le choc hémorragique, le choc septique, la maladie de Raynaud et les troubles de la fertilité.
- L'obésité; le diabète de type II, le syndrome métabolique, la résistance à l'insuline et l'inflammation du tissu adipeux.

Plus particulièrement, les composés de formule (I) selon la présente invention seront utiles pour la préparation de médicaments permettant la prévention et/ou le traitement de la douleur, des maladies inflammatoires, des maladies auto-immunes, des maladies allergiques, des maladies infectieuses, des maladies neuro dégénératives, des maladies cardiovasculaires, des cancers, des maladies gastro-intestinales, de l'obésité, du diabète de type II, de la résistance à l'insuline et de l'inflammation du tissu adipeux.

L'utilisation des composés selon l'invention pour la prévention et/ou le traitement des maladies ci-dessus mentionnées, ainsi que pour la préparation de médicaments destinés à traiter ces maladies fait partie intégrante de l'invention.

Les composés de formule (I) ci-dessus, ou l'un de leurs sels pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,1 à 4000 mg par jour, plus particulièrement de 0,5 à 1000 mg selon l'âge du sujet à traiter ou le type de traitement : prophylactique ou curatif.

Ainsi, selon un autre de ses aspects, la présente invention concerne des compositions pharmaceutiques renfermant, en tant que principe actif, un composé de formule (I), ou l'un de ses sels pharmaceutiquement acceptables ainsi qu'un ou plusieurs excipients pharmaceutiquement acceptables.

Dans les compositions pharmaceutiques de la présente invention pour l'administration par voie orale, sublinguale, inhalée, sous-cutanée, intramusculaire, intraveineuse, topique, locale, intratrachéale, intranasale, transdermique, locale ou rectale, les principes actifs peuvent être administrés sous formes unitaires d'administration, en mélange avec des supports pharmaceutiques classiques, aux animaux et aux êtres humains.

Les formes unitaires d'administration appropriées comprennent les formes par voie orale telles que les comprimés, les gélules molles ou dures, les poudres, les granules, les solutions ou suspensions orales, les formes d'administration sublinguale, buccale, intratrachéale, intraoculaire, intranasale, par inhalation, les aérosols, les formes d'administration topique, transdermique, les implants, les formes d'administration sous-cutanée, intramusculaire, intraveineuse et les formes d'administration rectale.

Pour l'administration topique on peut utiliser les composés selon l'invention dans des crèmes, des pommades, des gels, ou des lotions.

A titre d'exemple, une forme unitaire d'administration d'un composé selon l'invention sous forme de comprimé peut comprendre les composants suivants :

| | |
|---|---|
| Composé selon l'invention | : 50,0 mg |
| Mannitol | : 223,75 mg |
| Croscarmellose sodique | : 6,0 mg |
| Amidon de maïs | : 15,0 mg |
| Hydroxypropyl-méthylcellulose | : 2,25 mg |
| Stéarate de magnésium | : 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou d'un de ses sels pharmaceutiquement acceptables.

Les composés selon l'invention pourront également être utilisés pour la préparation de compositions à usage vétérinaire.

De plus, les composés selon l'invention, tel quel ou sous forme radiomarquée peuvent être utilisés comme outils pharmacologiques chez l'homme ou chez l'animal, pour la détection et la marquage des récepteurs aux cannabinoïdes CB₂.

## Revendications

1. Composé répondant à la formule (I) : 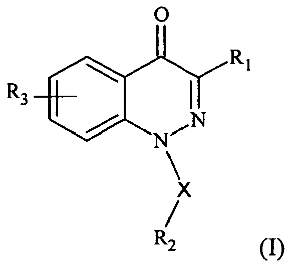 dans laquelle :
- X représente un radical bivalent (C₂-C₅)alkylène non substitué ou substitué une ou plusieurs fois par un groupe Alk ;
- R₁ représente :
un phényle substitué une ou plusieurs fois par des substituants choisis indépendamment parmi un atome d'halogène, un groupe Alk, un groupe Alk ;
un pyridyle substitué une ou plusieurs fois par des substituants choisis indépendamment parmi un atome d'halogène, un groupe Alk ;
- R₂ représente un atome d'hydrogène, un atome d'halogène, un groupe Alk, un groupe OAlk, un groupe -S-Alk, un groupe -SO-Alk, un groupe -SO₂Alk, un groupe -CO-N(R₄)-Alk, un groupe -N(R₄)SO₂-Alk, un groupe - N(R₄)CO-Alk, un groupe -N(R₄)-SO₂-N(Alk)₂ ;
- R₃ représente un atome d'hydrogène, un atome d'halogène, un groupe Alk ou un groupe Alk ;
- R₄ représente un atome d'hydrogène ou un (C₁-C₄)alkyle ;
- Alk représente un (C₁-C₄)alkyle non substitué ou substitué une ou plusieurs fois par un atome de fluor ;
à l'état de base ou de sel d'addition à un acide.

2. Composés de formule (I) selon la revendication 1 dans laquelle :
- X représente un radical bivalent (C₂-C₅)alkylène non substitué ou substitué une ou plusieurs fois par un méthyle ;
- R₁ représente : 3-fluoro-2-trifluorométhylphényle, 2-fluoro-3-trifluorométhylphényle, 3-trifluorométhoxyphényle, 2-chloro-3-trifluorométhylphényle, 3-trifluorométhylphényle, 5-fluoro-2-méthoxyphényle, 4-fluoro-2-méthoxyphényle, 2,3-dichlorophényle, 2-(trifluorométhyl)pyridin-3-yle ;
- R₂ représente un atome d'hydrogène, un atome de fluor, de chlore, un radical trifluorométhyle, un groupe -OCH₃, un groupe -OCH₂CH₃, un groupe -S-CH₃, un groupe -S-CH₂-CH₃, un groupe -SO-CH₂-CH₃, un groupe -SO₂CH₃, un groupe -SO₂CH₂CH₃, un groupe -NHSO₂CH₃, un groupe -NHSO₂-CF₃, un groupe -NHSO₂CHF₂, un groupe -N(CH₃)-SO₂CH₃, un groupe -NH-CO-CF₃, un groupe -N(CH₃)-CO-CF₃, un groupe -NH-SO₂-N(CH₃)₂, un groupe -CO-N(CH₃)₂, placés en position terminale de la chaîne alkyle ;
- R₃ représente un atome d'hydrogène, un atome de chlore, un groupe méthyle, un groupe méthoxy ou un radical trifluorométhyle ;
à l'état de base ou de sel d'addition à un acide.

3. Composé selon la revendication 1 de formule (I) choisi parmi :
- 3-[3-Fluoro-2-(trifluorométhyl)phényl]-8-méthoxy-1-pentylcinnolin-4(1*H*)-one ;
- 3-[2-Fluoro-3-(trifluorométhyl)phényl]-8-méthoxy-1-pentylcinnolin-4(1*H*)-one ;
- 3-[2-Fluoro-3-(trifluorométhyl)phényl]-1-pentylcinnolin-4(1*H*)-one ;
- 3-[2-Fluoro-3-(trifluorométhyl)phényl]-1-(5,5,5-trifluoropentyl)cinnolin-4(1*H*)-one ;
- 8-Méthoxy-1-pentyl-3-[3-(trifluorométhoxy)phényl]cinnolin-4(1*H*)-one ;
- 3-[2-Fluoro-3-(trifluorométhyl)phényl]-1-(4,4,4-trifluorobutyl)cinnolin-4(1*H*)-one ;
- 3-[2-Fluoro-3-(trifluorométhyl)phényl]-1-[3-(méthylthio)propyl]cinnolin-4(1*H*)-one ;
- 1-(4-Fluorobutyl)-3-[2-fluoro-3-(trifluorométhyl)phényl]cinnolin-4(1*H*)-one ;
- 3-[2-Chloro-3-(trifluorométhyl)phényl]-8-méthoxy-1-pentylcinnolin-4(1*H*)-one ;
- 3-[3-(Trifluorométhyl)phényl]-1-(5,5,5-trifluoropentyl)cinnolin-4(1*H*)-one ;
- 3-[2-Méthoxy-5-(trifluorométhyl)phényl]-1-(5,5,5-trifluoropentyl)cinnolin-4(1*H*)-one ;
- 1-(3-Chloropropyl)-3-[3-(trifluorométhyl)phényl]cinnolin-4(1*H*)-one ;
- 1-Pentyl-3-[3-(trifluorométhyl)phényl]cinnolin-4(1*H*)-one ;
- 1-[3-(Méthylthio)propyl]-3-[3-(trifluorométhyl)phényl]cinnolin-4(1*H*)-one ;
- 3-(4-Fluoro-2-méthoxyphényl)-1-(4,4,4-trifluorobutyl)cinnolin-4(1*H*)-one ;
- 3-(5-Fluoro-2-méthoxyphényl)-1-(4,4,4-trifluorobutyl)cinnolin-4(1*H*)-one ;
- 7-Chloro-3-[2-chloro-3-(trifluorométhyl)phényl]-1-(5,5,5-trifluoropentyl)cinnolin-4(1H)-one ;
- N-(3-[3-[2-fluoro-3-(trifluorométhyl)phényl]-8-méthoxy-4-oxocinnolin-1(4*H*)-yl]propyl)méthanesulfonamide ;
- N-[3-[3-[2-chloro-3-(trifluorométhyl)phényl]-8-méthoxy-4-oxocinnolin-1(4*H*)-yl]propyl]méthanesulfonamide ;
- N-[3-[3-[3-fluoro-2-(trifluorométhyl)phényl]-8-méthoxy-4-oxocinnolin-1(4*H*)-yl]propyl]méthanesulfonamide ;
- N-[3-[3-(2,3-dichlorophényl-8-méthoxy-4-oxocinnolin-1(4*H*)-yl)propyl]méthanesulfonamide ;
- N-[3-[8-méthoxy-4-oxo-3-[2-(trifluorométhyl)pyridin-3-yl]cinnolin-1(4*H*)-yl]propyl]méthanesulfonamide ;
- N-[3-[3-(2,3-dichlorophényl)-4-oxo-7-(trifluorométhyl)cinnolin-1(4*H*)-yl]propyl]méthanesulfonamide ;
- 3-[2-Fluoro-3-(trifluorométhyl)phényl]-1-(3-méthanesulfonyl-propyl)-8-méthoxy-1*H*-cinnolin-4-one ;
- N-{3-[3-[2-fluoro-3-(trifluorométhyl)-phényl]-4-oxo-7-trifluorométhyl-4*H-*cinnolin-1-yl]-propyl}-méthanesulfonamide ;
- N-{3-[3-[3-fluoro-2-(trifluorométhyl)-phényl]-4-oxo-7-trifluorométhyl-4*H-*cinnolin-1-yl]-propyl}-méthanesulfonamide ;
- N-{3-[3-[2-fluoro-3-(trifluorométhyl)-phényl]-4-oxo-4*H*-cinnolin-1-yl]-propyl}-méthanesulfonamide ;
- N-{3-[3-[2-fluoro-3-(trifluorométhyl)phényl]-7-méthoxy-4-oxo-4*H*-cinnolin-1-yl]-propyl}-méthanesulfonamide ;
- 1-[2-(Ethylthio)éthyl]-3-[2-fluoro-3-(trifluorométhyl)phényl]-8-méthoxycinnolin-4(1*H*)-one;
- 1-[2-(Ethylsulfinyl)éthyl]-3-[2-fluoro-3-(trifluorométhyl)phényl]-8-méthoxycinnolin-4(1*H*)-one ;
- 1-[2-(Ethylsulfonyl)éthyl]-3-[2-fluoro-3-(trifluorométhyl)phényl]-8-méthoxycinnolin-4(1*H*)-one ;
- 3-(2,3-Dichlorophényl)-1-[2-(éthylthio)éthyl]-8-méthoxycinnolin-4(1*H*)-one;
- 3-(2,3-Dichlorophényl)-1-[2-(éthylsulfinyl)éthyl]-8-méthoxycinnolin-4(1*H*)-one ;
- 3-(2,3-Dichlorophényl)-1-[2-(éthylsulfonyl)éthyl]-8-méthoxycinnolin-4(1*H*)-one ;
- 1,1,1-Trifluoro-N-(3-{3-[2-fluoro-3-(trifluorométhyl)phényl]-8-méthoxy-4-oxocinnolin-1(4*H*)-yl}propyl)méthanesulfonamide ;
- 2,2,2-Trifluoro-N-(3-{3-[2-fluoro-3-(trifluorométhyl)phényl]-8-méthoxy-4-oxocinnolin-1(4*H*)-yl}propyl)acétamide ;
- N-(3-{7-chloro-3-[2-fluoro-3-(trifluorométhyl)phényl]-4-oxocinnolin-1(4*H*)-yl}propyl)méthanesulfonamide ;
- N-{3-[3-(2,3-dichlorophényl)-8-méthoxy-4-oxocinnolin-1(4*H*)-yl]propyl -N-méthylméthanesulfonamide ;
- 1-[2-(Ethylthio)éthyl]-3-[2-fluoro-3-(trifluorométhyl)phényl]-8-(trifluorométhoxy)cinnolin-4(1*H*)-one ;
- 7-Chloro-1-[2-(éthylthio)éthyl]-3-[2-fluoro-3-(trifluorométhyl)phényl]cinnolin-4(1*H*)-one ;
- 7-Chloro-1-[2-(éthylsulfonyl)éthyl]-3-[2-fluoro-3-(trifluorométhyl)phényl]cinnolin-4(1*H*)-one ;
- 7-Chloro-3-(2,3-dichlorophényl)-1-[2-(éthylthio)éthyl]cinnolin-4(1*H*)-one ;
- 2,2,2-Trifluoro-N-(3-{3-[2-fluoro-3-(trifluorométhyl)phényl]-8-méthoxy-4-oxocinnolin-1(4*H*)-yl}propyl)-N-méthylacétamide ;
- N-(3-{7-chloro-3-[3-fluoro-2-(trifluorométhyl)phényl]-4-oxocinnolin-1(4*H*)-yl}propyl)méthanesulfonamide ;
- 7-chloro-1-[2-(éthylthio)éthyl]-3-[3-(trifluorométhoxy)phényl] cinnolin-4(1*H*)-one ;
- 7-Chloro-3-(2,3-dichlorophényl)-1-[2-(éthylsulfinyl)éthyl]cinnolin-4(1*H*)-one ;
- 7-Chloro-3-(2,3-dichlorophényl)-1-[2-(éthylsulfonyl)éthyl]cinnolin-4(1*H*)-one ;
- 1-[2-(Ethylthio)éthyl]-8-méthoxy-3-[3-(trifluorométhoxy)phényl]cinnolin-4(1*H*)-one ;
- 1-[2-(éthylsulfonyl)éthyl]-8-méthoxy-3-[3-(trifluorométhoxy)phényl]cinnolin-4(1*H*)-one ;
- 3-(2,3-dichlorophényl)-1-(2-éthoxyéthyl)-8-méthoxycinnolin-4(1*H*)-one ;
- 1-(2-éthoxyéthyl)-3-[2-fluoro-3-(trifluorométhyl)phényl]-8-méthoxycinnolin-4(1*H*)-one ;
- N'-{3-[3-(2,3-dichlorophényl)-8-méthoxy-4-oxocinnolin-1(4*H*)-yl]propyl}-N,N-diméthylsulfamide ;
- 1,1-difluoro-N-(3-{3-[2-fluoro-3-(trifluorométhyl)phényl]-8-méthoxy-4-oxocinnolin-1(4*H*)-yl}propyl)méthanesulfonamide
- N-(2-{3-[2-fluoro-3-(trifluorométhyl)phényl]-8-méthoxy-4-oxocinnolin-1(4*H*)-yl}éthyl)méthanesulfonamide ;
- 3-{3-[2-fluoro-3-(trifluorométhyl)phényl]-8-méthoxy-4-oxocinnolin-1(4*H*)-yl}-N,N-diméthylpropanamide ;
- 3-[2-fluoro-3-(trifluorométhyl)phényl]-8-méthoxy-1-(3-méthoxy-3-méthylbutyl)cinnolin-4(1*H*)-one ;
- 3-(2,3-dichlorophényl)-8-méthoxy-1-(3-méthoxy-3-méthylbutyl)cinnolin-4(1*H*)-one ;
- 3-[3-fluoro-2-(trifluorométhyl)phényl]-8-méthoxy-1-(3-méthoxy-3-méthylbutyl)cinnolin-4(1*H*)-one ;
- 8-fluoro-3-[2-fluoro-3-(trifluorométhyl)phényl]-1-(3-méthoxy-3-méthylbutyl)cinnolin-4(1*H*)-one ;
à l'état de base ou de sel d'addition à un acide.

4. Procédé de préparation des composés de formule (I) selon l'une quelconque des revendications 1 à 3, **caractérisé en ce que** :
On fait réagir en présence d'une base, un composé de formule : 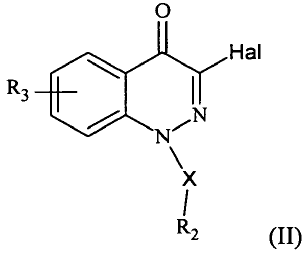 dans laquelle X, R₂ et R₃ sont tels que définis pour un composé de formule (I) à la revendication 1, et Hal représente un atome d'halogène avec un composé de formule :
R₁-B(OH)₂ (III)
dans laquelle R₁ est tel que défini pour un composé de formule (I) à la revendication 1.

5. Procédé de préparation des composés de formule (I) dans laquelle R₂ représente un groupe -S-Alk, selon l'une quelconque des revendications 1 à 3, **caractérisé en ce que** :
on fait réagir en présence d'une base, un composé de formule : 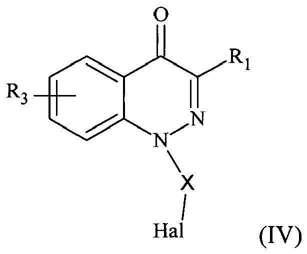 dans laquelle X, R₁ et R₃ sont tels que définis pour un composé de formule (I) à la revendication 1 et Hal représente un atome d'halogène, avec un dérivé d'alcanethiolate de sodium de formule:
NaS-Alk (V)

6. Médicament, **caractérisé en ce qu'**il contient un composé de formule (I) selon l'une quelconque des revendications 1 à 3, ou encore un sel pharmaceutiquement acceptable du composé de formule (I).

7. Composition pharmaceutique, **caractérisé en ce qu'**elle comprend un composé de formule (I) selon l'une quelconque des revendications 1 à 3, un sel pharmaceutiquement acceptable de ce composé, ainsi qu'au moins un excipient pharmaceutiquement acceptable.

8. Utilisation d'un composé de formule (I) selon l'une quelconque des revendications 1 à 3 pour la préparation d'un médicament destiné à traiter et à prévenir la douleur, les maladies inflammatoires, les maladies auto-immunes, les maladies allergiques, les maladies infectieuses, les maladies neurogénératives, les maladies cardiovasculaires, les cancers, les maladies gastro-intestinales, l'obésité, le diabète de type II, la résistance à l'insuline et l'inflammation du tissu adipeux.

## Patentansprüche

1. Verbindung der Formel (I): 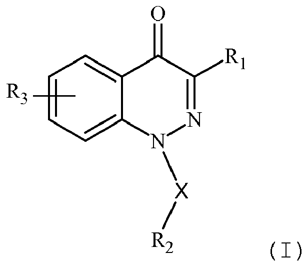 worin:
- X für einen zweiwertigen (C₂-C₅)-Alkylenrest, der unsubstituiert oder ein- oder mehrfach durch eine Alk-Gruppe substituiert ist, steht;
- R₁ für:
. ein Phenyl, das ein- oder mehrfach durch Substituenten, die unabhängig voneinander aus einem Halogenatom, einer Alk-Gruppe und einer OAlk-Gruppe ausgewählt sind, substituiert ist;
. ein Pyridyl, das ein- oder mehrfach durch Substituenten, die unabhängig voneinander aus einem Halogenatom und einer Alk-Gruppe ausgewählt sind, substituiert ist;
steht;
- R₂ für ein Wasserstoffatom, ein Halogenatom, eine Alk-Gruppe, eine -OAlk-Gruppe, eine -S-Alk-Gruppe, eine -SO-Alk-Gruppe, eine -SO₂-Alk-Gruppe, eine-CO-N(R₄)-Alk-Gruppe, eine -N (R₄)SO₂-Alk-Gruppe, eine -N(R₄)CO-Alk-Gruppe oder eine -N(R₄)-SO₂-N(Alk)₂-Gruppe steht;
- R₃ für ein Wasserstoffatom, ein Halogenatom, eine Alk-Gruppe oder eine OAlk-Gruppe steht;
- R₄ für ein Wasserstoffatom oder ein (C₁-C₄)-Alkyl steht;
- Alk für ein (C₁-C₄)-Alkyl, das unsubstituiert oder ein- oder mehrfach durch ein Fluoratom substituiert ist, steht;
in Basen- oder Säureadditionssalzform.

2. Verbindung der Formel (I) nach Anspruch 1, worin:
- X für einen zweiwertigen (C₂-C₅)-Alkylenrest, der unsubstituiert oder ein- oder mehrfach durch Methyl substituiert ist, steht;
- R₁ für: 3-Fluor-2-trifluormethylphenyl, 2-Fluor-3-trifluoromethylphenyl, 3-Trifluormethoxyphenyl, 2-Chlor-3-trifluormethylphenyl, 3-Trifluoromethylphenyl, 5-Fluor-2-methoxyphenyl, 4-Fluor-2-methoxyphenyl, 2,3-Dichlorphenyl oder 2-(Trifluormethyl)pyridin-3-yl steht;
- R₂ für ein Wasserstoffatom, ein Fluor- oder Chloratom, einen Trifluormethylrest, eine -OCH₃-Gruppe, eine -OCH₂CH₃-Gruppe, eine -S-CH₃-Gruppe, eine -S-CH₂-CH₃-Gruppe, eine -SO-CH₂-CH₃-Gruppe, eine -SO₂CH₃-Gruppe, eine -SO₂CH₂CH₃-Gruppe, eine -NHSO₂CH₃-Gruppe, eine -NHSO₂-CF₃-Gruppe, eine -NHSO₂CHF₂-Gruppe, eine -N(CH₃)-SO₂CH₃-Gruppe, eine -NH-CO-CF₃-Gruppe, eine -N(CH₃)-CO-CF₃-Gruppe, eine -NH-SO₂-N(CH₃)₂-Gruppe oder eine -CO-N(CH₃)₂-Gruppe, die in der endständigen Position der Alkylkette stehen, steht;
- R₃ für ein Wasserstoffatom, ein Chloratom, eine Methylgruppe, eine Methoxygruppe oder einen Trifluormethylrest steht;
in Basen- oder Säureadditionssalzform.

3. Verbindung nach Anspruch 1 der Formel (I), ausgewählt aus:
- 3-[3-Fluor-2-(trifluormethyl)phenyl]-8-methoxy-1-pentylcinnolin-4(1*H*)-on;
- 3-[2-Fluor-3-(trifluormethyl)phenyl]-8-methoxy-1-pentylcinnolin-4(1*H*)-on;
- 3-[2-Fluor-3-(trifluormethyl)phenyl]-1-pentyl-cinnolin-4(1*H*)-on;
- 3-[2-Fluor-3-(trifluormethyl)phenyl]-1-(5,5,5-trifluorpentyl)cinnolin-4(1*H*)-on;
- 8-Methoxy-1-pentyl-3-[3-(trifluormethoxy)-phenyl]cinnolin-4(1*H*)-on;
- 3-[2-Fluor-3-(trifluormethyl)phenyl]-1-(4,4,4-trifluorbutyl)cinnolin-4(1*H*)-on;
- 3-[2-Fluor-3-(trifluormethyl)phenyl]-1-[3-(methylthio)propyl]cinnolin-4(1*H*)-on;
- 1-(4-Fluorbutyl)-3-[2-fluor-3-(trifluormethyl)-phenyl]cinnolin-4(1*H*)-on;
- 3-[2-Chlor-3-(trifluormethyl)phenyl]-8-methoxy-1-pentylcinnolin-4(1*H*)-on;
- 3-[3-(Trifluormethyl)phenyl]-1-(5,5,5-trifluor-pentyl)cinnolin-4(1*H*)-on;
- 3-[2-Methoxy-5-(trifluormethyl)phenyl]-1-(5,5,5-trifluorpentyl)cinnolin-4(1*H*)-on;
- 1-(3-Chlorpropyl)-3-[3-(trifluormethyl)phenyl]-cinnolin-4(1*H*)-on;
- 1-Pentyl-3-[3-(trifluormethyl)phenyl]cinnolin-4(1*H*)-on;
- 1-[3-(Methylthio)propyl]-3-[3-(trifluormethyl)-phenyl]cinnolin-4(1*H*)-on;
- 3-(4-Fluor-2-methoxyphenyl)-1-(4,4,4-trifluor-butyl)cinnolin-4(1*H*)-on;
- 3-(5-Fluor-2-methoxyphenyl)-1-(4,4,4-trifluor-butyl)cinnolin-4(1*H*)-on;
- 7-Chlor-3-[2-chlor-3-(trifluormethyl)phenyl]-1-(5,5,5-trifluorpentyl)cinnolin-4(1*H*)-on;
- N-(3-[3-[2-Fluor-3-(trifluormethyl)phenyl]-8-methoxy-4-oxocinnolin-1(4*H*)-yl]propyl)methan-sulfonamid;
- N-[3-[3-[2-Chlor-3-(trifluormethyl)phenyl]-8-methoxy-4-oxocinnolin-1(4*H*)-yl]propyl]methansulfonamid;
- N-[3-[3-[3-Fluor-2-(trifluormethyl)phenyl]-8-methoxy-4-oxocinnolin-1(4*H*)-yl]propyl]methansulfonamid;
- N-[3-[3-(2,3-Dichlorphenyl)-8-methoxy-4-oxocinnolin-1(4*H*)-yl]propyl]methansulfonamid;
- N-[3-[8-Methoxy-4-oxo-3-[2-(trifluormethyl)-pyridin-3-yl]cinnolin-1(4*H*)-yl]propyl]methansulfonamid;
- N-[3-[3-(2,3-Dichlorphenyl)-4-oxo-7-(trifluormethyl)cinnolin-1(4*H*)-yl]propyl]methansulfonamid;
- 3-[2-Fluor-3-(trifluormethyl)phenyl]-1-(3-(methansulfonyl)propyl)-8-methoxy-1*H*-cinnolin-4-on;
- N-{3-[3-[2-Fluor-3-(trifluormethyl)phenyl]-4-oxo-7-trifluormethyl-4*H*-cinnolin-1-yl]propyl}-methansulfonamid;
- N-{3-[3-[3-Fluor-2-(trifluormethyl)phenyl]-4-oxo-7-trifluormethyl-4*H*-cinnolin-1-yl]propyl}-methansulfonamid;
- N-{3-[3-[2-Fluor-3-(trifluormethyl)phenyl]-4-oxo-4*H*-cinnolin-1-yl]-propyl}methansulfonamid;
- N-{3-[3-[2-Fluor-3-(trifluormethyl)phenyl]-7-methoxy-4-oxo-4*H*-cinnolin-1-yl]propyl}methansulfonamid;
- 1-[2-(Ethylthio)ethyl]-3-[2-fluor-3-(trifluormethyl)phenyl]-8-methoxycinnolin-4(1*H*)-on;
- 1-[2-(Ethylsulfinyl)ethyl]-3-[2-fluor-3-(trifluormethyl)phenyl]-8-methoxycinnolin-4(1*H*)-on;
- 1-[2-(Ethylsulfonyl)ethyl]-3-[2-fluor-3-(trifluormethyl)phenyl]-8-methoxycinnolin-4(1*H*)-on;
- 3-(2,3-Dichlorphenyl)-1-[2-(ethylthio)ethyl]-8-methoxycinnolin-4(1*H*)-on;
- 3-(2,3-Dichlorphenyl)-1-[2-(ethylsulfinyl)-ethyl]-8-methoxycinnolin-4(1*H*)-on;
- 3-(2,3-Dichlorphenyl)-1-[2-(ethylsulfonyl)-ethyl]-8-methoxycinnolin-4(1*H*)-on;
- 1,1,1-Trifluor-N-(3-{3-[2-fluor-3-(trifluormethyl)phenyl]-8-methoxy-4-oxocinnolin-1(4*H*)-yl}propyl)methansulfonamid;
- 2,2,2-Trifluor-N-(3-{3-[2-fluor-3-(trifluor-methyl)phenyl]-8-methoxy-4-oxocinnolin-1(4*H*)-yl}propyl)acetamid;
- N-(3-{7-Chlor-3-[2-fluor-3-(trifluormethyl)-phenyl]-4-oxocinnolin-1(4*H*)-yl}propyl)methansulfonamid;
- N-{3-[3-(2,3-Dichlorphenyl)-8-methoxy-4-oxocinnolin-1(4*H*)-yl]propyl}-N-methylmethansulfon-amid;
- 1-[2-(Ethylthio)ethyl]-3-[2-fluor-3-(trifluormethyl)phenyl]-8-(trifluormethoxy)cinnolin-4(1*H*)-on;
- 7-Chlor-1-[2-(ethylthio)ethyl]-3-[2-fluor-3-(trifluormethyl)phenyl]cinnolin-4(1*H*)-on;
- 7-Chlor-1-[2-(ethylsulfonyl)ethyl]-3-[2-fluor-3-(trifluormethyl)phenyl]cinnolin-4(1*H*)-on;
- 7-Chlor-3-(2,3-dichlorphenyl)-1-[2-(ethylthio)-ethyl]cinnolin-4(1*H*)-on;
- 2,2,2-Trifluor-N-(3-{3-[2-fluor-3-(trifluor-methyl)phenyl]-8-methoxy-4-oxocinnolin-1(4*H*)-yl}propyl)-N-methylacetamid;
- N-(3-{7-Chlor-3-[3-fluor-2-(trifluormethyl)-phenyl]-4-oxocinnolin-1(4*H*)-yl}propyl)methansulfonamid;
- 7-Chlor-1-[2-(ethylthio)ethyl]-3-[3-(trifluor-methoxy)phenyl]cinnolin-4(1*H*)-on;
- 7-Chlor-3-(2,3-dichlorphenyl)-1-[2-(ethyl-sulfinyl)ethyl]cinnolin-4(1*H*)-on;
- 7-Chlor-3-(2,3-dichlorphenyl)-1-[2-(ethylsulfonyl)ethyl]cinnolin-4(1*H*)-on;
- 1-[2-(Ethylthio)ethyl]-8-methoxy-3-[3-(trifluor-methoxy)phenyl]cinnolin-4(1*H*)-on;
- 1-[2-(Ethylsulfonyl)ethyl]-8-methoxy-3-[3-(tri-fluormethoxy)phenyl]cinnolin-4(1*H*)-on;
- 3-(2,3-Dichlorphenyl)-1-(2-ethoxyethyl)-8-methoxycinnolin-4(1*H*)-on;
- 1-(2-Ethoxyethyl)-3-[2-fluor-3-(trifluormethyl)-phenyl]-8-methoxycinnolin-4(1*H*)-on;
- N'-{3-[3-(2,3-Dichlorphenyl)-8-methoxy-4-oxo-cinnolin-1(4*H*)-yl]propyl}-N,N-dimethylsulfamid;
- 1,1-Difluor-N-(3-{3-[2-fluor-3-(trifluormethyl)-phenyl]-8-methoxy-4-oxocinnolin-1(4*H*)-yl}propyl)-methansulfonamid;
- N-(2-{3-[2-Fluor-3-(trifluormethyl)phenyl]-8-methoxy-4-oxocinnolin-1(4*H*)-yl}ethyl)methansulfonamid;
- 3-{3-[2-Fluor-3-(trifluormethyl)phenyl]-8-methoxy-4-oxocinnolin-1(4*H*)-yl}-N,N-dimethyl-propanamid;
- 3-[2-Fluor-3-(trifluormethyl)phenyl]-8-methoxy-1-(3-methoxy-3-methylbutyl)cinnolin-4(1*H*)-on;
- 3-(2,3-Dichlorphenyl)-8-methoxy-1-(3-methoxy-3-methylbutyl)cinnolin-4(1*H*)-on;
- 3-[3-Fluor-2-(trifluormethyl)phenyl]-8-methoxy-1-(3-methoxy-3-methylbutyl)cinnolin-4(1*H*)-on;
- 8-Fluor-3-[2-fluor-3-(trifluormethyl)phenyl]-1-(3-methoxy-3-methylbutyl)cinnolin-4(1*H*)-on;
in Basen- oder Säureadditionssalzform.

4. Verfahren zur Herstellung einer Verbindung der Formel (I) nach einem der Ansprüche 1 bis 3, **dadurch gekennzeichnet, dass**:
man eine Verbindung der Formel: 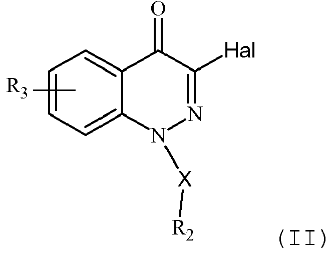 worin X, R₂ und R₃ wie für eine Verbindung der Formel (I) in Anspruch 1 definiert sind und Hal für ein Halogenatom steht, in Gegenwart einer Base mit einer Verbindung der Formel:
R₁-B(OH)₂ (III)
worin R₁ wie für eine Verbindung der Formel (I) in Anspruch 1 definiert ist, umsetzt.

5. Verfahren zur Herstellung einer Verbindung der Formel (I), worin R₂ für eine -S-Alk-Gruppe steht, nach einem der Ansprüche 1 bis 3, **dadurch gekennzeichnet, dass**:
man eine Verbindung der Formel: 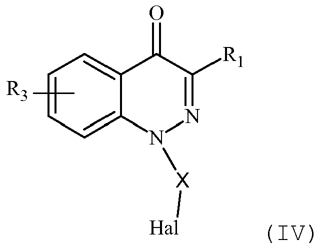 worin X, R₁ und R₃ wie für eine Verbindung der Formel (I) in Anspruch 1 definiert sind und Hal für ein Halogenatom steht, in Gegenwart einer Base mit einem Natriumalkanthiolatderivat der Formel:
NaS-Alk (V)

6. Medikament, **dadurch gekennzeichnet, dass** es eine Verbindung der Formel (I) nach einem der Ansprüche 1 bis 3 oder ein pharmazeutisch unbedenkliches Salz der Verbindung der Formel (I) enthält.

7. Pharmazeutische Zusammensetzung, **dadurch gekennzeichnet, dass** sie eine Verbindung der Formel (I) nach einem der Ansprüche 1 bis 3, ein pharmazeutisch unbedenkliches Salz dieser Verbindung sowie mindestens einen pharmazeutisch unbedenklichen Hilfsstoff umfasst.

8. Verwendung einer Verbindung der Formel (I) nach einem der Ansprüche 1 bis 3 zur Herstellung eines Medikaments zur Behandlung und Prävention von Schmerzen, entzündlichen Erkrankungen, Autoimmunerkrankungen, allergischen Erkrankungen, Infektionserkrankungen, neurodegenerativen Erkrankungen, Herz-Kreislauf-Erkrankungen, Krebserkrankungen, Magen-Darm-Erkrankungen, Obesitas, Typ-II-Diabetes, Insulinresistenz und Fettgewebeentzündung.

## Claims

1. Compound corresponding to the formula (I): 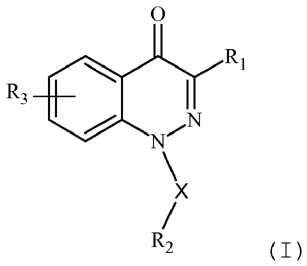 in which:
- X represents a divalent (C₂-C₅)alkylene radical which is unsubstituted or substituted one or more times by an Alk group;
- R₁ represents:
. a phenyl which is substituted one or more times by substituents chosen independently from a halogen atom, an Alk group or an OAlk group;
. a pyridyl which is substituted one or more times by substituents chosen independently from a halogen atom or an Alk group;
- R₂ represents a hydrogen atom, a halogen atom, an Alk group, an OAlk group, an -S-Alk group, an -SO-Alk group, an -SO₂-Alk group, a -CO-N(R₄)-Alk group, an -N(R₄)SO₂-Alk group, an -N(R₄)CO-Alk group or an -N(R₄)-SO₂-N(Alk)₂ group;
- R₃ represents a hydrogen atom, a halogen atom, an Alk group or an OAlk group;
- R₄ represents a hydrogen atom or a (C₁-C₄)alkyl;
- Alk represents a (C₁-C₄)alkyl which is unsubstituted or substituted one or more times by a fluorine atom;
in the form of the base or of an addition salt with an acid.

2. Compound of formula (I) according to Claim 1, in which:
- X represents a divalent (C₂-C₅)alkylene radical which is unsubstituted or substituted one or more times by a methyl;
- R₁ represents: 3-fluoro-2-trifluoromethylphenyl, 2-fluoro-3-trifluoromethylphenyl, 3-trifluoromethoxyphenyl, 2-chloro-3-trifluoromethylphenyl, 3-trifluoromethylphenyl, 5-fluoro-2-methoxyphenyl, 4-fluoro-2-methoxyphenyl, 2,3-dichlorophenyl or 2-(trifluoromethyl)pyridin-3-yl;
- R₂ represents a hydrogen atom, a fluorine or chlorine atom, a trifluoromethyl radical, an - OCH₃ group, an -OCH₂CH₃ group, an -S-CH₃ group, an -S-CH₂-CH₃ group, an -SO-CH₂-CH₃ group, an - SO₂CH₃ group, an -SO₂CH₂CH₃ group, an -NHSO₂-CH₃ group, an -NHSO₂-CF₃ group, an -NHSO₂CHF₂ group, an -N(CH₃)-SO₂CH₃ group, an -NH-CO-CF₃ group, an
- N(CH₃)-CO-CF₃ group, an -NH-SO₂-N(CH₃)₂ group or a -CO-N(CH₃)₂ group, which are placed on the terminal position of the alkyl chain;
- R₃ represents a hydrogen atom, a chlorine atom, a methyl group, a methoxy group or a trifluoromethyl radical;
in the form of the base or of an addition salt with an acid.

3. Compound according to Claim 1 of formula (I) chosen from:
- 3-[3-Fluoro-2-(trifluoromethyl)phenyl]-8-methoxy-1-pentylcinnolin-4(1*H*)-one;
- 3-[2-Fluoro-3-(trifluoromethyl)phenyl]-8-methoxy-1-pentylcinnolin-4(1*H*)-one;
- 3-[2-Fluoro-3-(trifluoromethyl)phenyl]-1-pentylcinnolin-4(1*H*)-one;
- 3-[2-Fluoro-3-(trifluoromethyl)phenyl]-1-(5,5,5-trifluoropentyl)cinnolin-4(1*H*)-one;
- 8-Methoxy-1-pentyl-3-[3-(trifluoromethoxy)phenyl]cinnolin-4(1*H*)-one;
- 3-[2-Fluoro-3-(trifluoromethyl)phenyl]-1-(4,4,4-trifluorobutyl)cinnolin-4(1*H*)-one;
- 3-[2-Fluoro-3-(trifluoromethyl)phenyl]-1-[3-(methylthio)propyl]cinnolin-4(1*H*)-one;
- 1-(4-Fluorobutyl)-3-[2-fluoro-3-(trifluoromethyl)phenyl]cinnolin-4(1*H*)-one;
- 3-[2-Chloro-3-(trifluoromethyl)phenyl]-8-methoxy-1-pentylcinnolin-4(1*H*)-one;
- 3-[3-(Trifluoromethyl)phenyl]-1-(5,5,5-trifluoropentyl)cinnolin-4(1*H*)-one;
- 3-[2-Methoxy-5-(trifluoromethyl)phenyl]-1-(5,5,5-trifluoropentyl)cinnolin-4(1*H*)-one;
- 1-(3-Chloropropyl)-3-[3-(trifluoromethyl)phenyl]cinnolin-4(1*H*)-one;
- 1-Pentyl-3-[3-(trifluoromethyl)phenyl]cinnolin-4(1*H*)-one;
- 1-[3-(Methylthio)propyl]-3-[3-(trifluoromethyl)phenyl]cinnolin-4(1*H*)-one;
- 3-(4-Fluoro-2-methoxyphenyl)-1-(4,4,4-trifluorobutyl)cinnolin-4(1*H*)-one;
- 3-(5-Fluoro-2-methoxyphenyl)-1-(4,4,4-trifluorobutyl)cinnolin-4(1*H*)-one;
- 7-Chloro-3-[2-chloro-3-(trifluoromethyl)phenyl]-1-(5,5,5-trifluoropentylcinnolin-4(1*H*)-one;
- N-(3-[3-[2-Fluoro-3-(trifluoromethyl)phenyl]-8-methoxy-4-oxocinnolin-1(4*H*)-yl]propyl)methanesulphonamide;
- N-[3-[3-[2-Chloro-3-(trifluoromethyl)phenyl]-8-methoxy-4-oxocinnolin-1(4*H*)-yl]propyl]methanesulphonamide;
- N-[3-[3-[3-Fluoro-2-(trifluoromethyl)phenyl]-8-methoxy-4-oxocinnolin-1(4*H*)-yl]propyl]methanesulphonamide;
- N-[3-[3-(2,3-Dichlorophenyl)-8-methoxy-4-oxocinnolin-1(4*H*)-yl]propyl]methanesulphonamide;
- N-[3-[8-Methoxy-4-oxo-3-[2-(trifluoromethyl)pyridin-3-yl]cinnolin-1(4*H*)-yl]propyl]methanesulphonamide;
- N-[3-[3-(2,3-Dichlorophenyl)-4-oxo-7-(trifluoromethyl)cinnolin-1(4*H*)-yl]propyl]methanesulphonamide;
- 3-[2-Fluoro-3-(trifluoromethyl)phenyl]-1-(3-(methanesulphonyl)propyl)-8-methoxy-1*H*-cinnolin-4-one;
- N-{3-[3-[2-Fluoro-3-(trifluoromethyl)phenyl]-4-oxo-7-trifluoromethyl-4*H*-cinnolin-1-yl]propyl}methanesulphonamide;
- N-{3-[3-[3-Fluoro-2-(trifluoromethyl)phenyl]-4-oxo-7-trifluoromethyl-4*H*-cinnolin-1-yl]propyl}methanesulphonamide;
- N-{3-[3-[2-Fluoro-3-(trifluoromethyl)phenyl]-4-oxo-4*H*-cinnolin-1-yl]-propyl}methanesulphonamide;
- N-{3-[3-[2-Fluoro-3-(trifluoromethyl)phenyl]-7-methoxy-4-oxo-4*H*-cinnolin-1-yl]propyl}methanesulphonamide;
- 1-[2-(Ethylthio)ethyl]-3-[2-fluoro-3-(trifluoromethyl)phenyl]-8-methoxycinnolin-4(1*H*)-one;
- 1-[2-(Ethylsulphinyl)ethyl]-3-[2-fluoro-3-(trifluoromethyl)phenyl]-8-methoxycinnolin-4(1*H*)-one;
- 1-[2-(Ethylsulphonyl)ethyl]-3-[2-fluoro-3-(trifluoromethyl)phenyl]-8-methoxycinnolin-4(1*H*)-one;
- 3-(2,3-Dichlorophenyl)-1-[2-(ethylthio)ethyl]-8-methoxycinnolin-4(1*H*)-one;
- 3-(2,3-Dichlorophenyl)-1-[2-(ethylsulphinyl)ethyl]-8-methoxycinnolin-4(1*H*)-one;
- 3-(2,3-Dichlorophenyl)-1-[2-(ethylsulphonyl)ethyl]-8-methoxycinnolin-4(1*H*)-one;
- 1,1,1-Trifluoro-N-(3-{3-[2-fluoro-3-(trifluoromethyl)phenyl]-8-methoxy-4-oxocinnolin-1(4*H*)-yl}propyl)methanesulphonamide;
- 2,2,2-Trifluoro-N-(3-{3-[2-fluoro-3-(trifluoromethyl)phenyl]-8-methoxy-4-oxocinnolin-1(4*H*)-yl}propyl)acetamide;
- N-(3-{7-Chloro-3-[2-fluoro-3-(trifluoromethyl)phenyl]-4-oxocinnolin-1(4*H*)-yl}propyl)methanesulphonamide;
- N-{3-[3-(2,3-Dichlorophenyl)-8-methoxy-4-oxocinnolin-1(4*H*)-yl]propyl}-N-methylmethanesulphonamide;
- 1-[2-(Ethylthio)ethyl]-3-[2-fluoro-3-(trifluoromethyl)phenyl]-8-(trifluoromethoxy)cinnolin-4(1*H*)-one;
- 7-Chloro-1-[2-(ethylthio)ethyl]-3-[2-fluoro-3-(trifluoromethyl)phenyl]cinnolin-4(1*H*)-one;
- 7-Chloro-1-[2-(ethylsulphonyl)ethyl]-3-[2-fluoro-3-(trifluoromethyl)phenyl]cinnolin-4(1*H*)-one;
- 7-Chloro-3-(2,3-dichlorophenyl)-1-[2-(ethylthio)ethyl]cinnolin-4(1*H*)-one;
- 2,2,2-Trifluoro-N-(3-{3-[2-fluoro-3-(trifluoromethyl)phenyl]-8-methoxy-4-oxocinnolin-1(4*H*)-yl}propyl)-N-methylacetamide;
- N-(3-{7-Chloro-3-[3-fluoro-2-(trifluoromethyl)phenyl]-4-oxocinnolin-1(4*H*)-yl}propyl)methanesulphonamide;
- 7-Chloro-1-[2-(ethylthio)ethyl]-3-[3-(trifluoromethoxy)phenyl]cinnolin-4(1*H*)-one;
- 7-Chloro-3-(2,3-dichlorophenyl)-1-[2-(ethylsulphinyl)ethyl]cinnolin-4(1*H*)-one;
- 7-Chloro-3-(2,3-dichlorophenyl)-1-[2-(ethylsulphonyl)ethyl]cinnolin-4(1*H*)-one;
- 1-[2-(Ethylthio)ethyl]-8-methoxy-3-[3-(trifluoromethoxy)phenyl]cinnolin-4(1*H*)-one;
- 1-[2-(Ethylsulphonyl)ethyl]-8-methoxy-3-[3-(trifluoromethoxy)phenyl]cinnolin-4(1*H*)-one;
- 3-(2,3-Dichlorophenyl)-1-(2-ethoxyethyl)-8-methoxycinnolin-4(1*H*)-one;
- 1-(2-Ethoxyethyl)-3-[2-fluoro-3-(trifluoromethyl)phenyl]-8-methoxycinnolin-4(1*H*)-one;
- N'-{3-[3-(2,3-Dichlorophenyl)-8-methoxy-4-oxocinnolin-1(4*H*)-yl]propyl}-N,N-dimethylsulphamide;
- 1,1-Difluoro-N-(3-{3-[2-fluoro-3-(trifluoromethyl)phenyl]-8-methoxy-4-oxocinnolin-1(4*H*)-yl}propyl)methanesulphonamide;
- N-(2-{3-[2-Fluoro-3-(trifluoromethyl)phenyl]-8-methoxy-4-oxocinnolin-1(4*H*)-yl}ethyl)methanesulphonamide;
- 3-{3-[2-Fluoro-3-(trifluoromethyl)phenyl]-8-methoxy-4-oxocinnolin-1(4*H*)-yl}-N,N-dimethylpropanamide;
- 3-[2-Fluoro-3-(trifluoromethyl)phenyl]-8-methoxy-1-(3-methoxy-3-methylbutyl)cinnolin-4(1*H*)-one;
- 3-(2,3-Dichlorophenyl)-8-methoxy-1-(3-methoxy-3-methylbutyl)cinnolin-4(1*H*)-one;
- 3-[3-Fluoro-2-(trifluoromethyl)phenyl]-8-methoxy-1-(3-methoxy-3-methylbutyl)cinnolin-4(1*H*)-one;
- 8-Fluoro-3-[2-fluoro-3-(trifluoromethyl)phenyl]-1-(3-methoxy-3-methylbutyl)cinnolin-4(1*H*)-one;
in the form of the base or of an addition salt with an acid.

4. Process for the preparation of a compound of formula (I) according to any one of Claims 1 to 3, **characterized in that**:
a compound of formula: 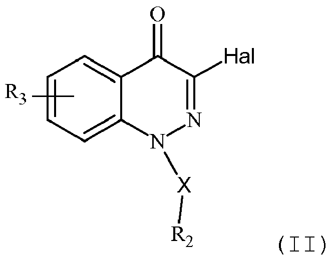 in which X, R₂ and R₃ are as defined for a compound of formula (I) in Claim 1 and Hal represents a halogen atom, is reacted in the presence of a base with a compound of formula:
**R₁-B(OH)₂** **(III)**
in which R₁ is as defined for a compound of formula (I) in Claim 1.

5. Process for the preparation of a compound of formula (I) in which R₂ represents an -S-Alk group according to any one of Claims 1 to 3, **characterized in that**:
a compound of formula: 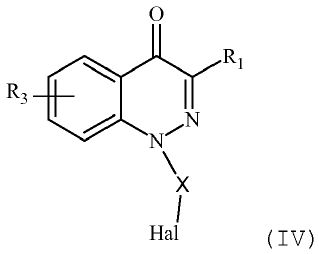 in which X, R₁ and R₃ are as defined for a compound of formula (I) in Claim 1 and Hal represents a halogen atom, is reacted in the presence of a base with a sodium alkanethiolate derivative of formula:
NaS-Alk (V)

6. Medicament, **characterized in that** it comprises a compound of formula (I) according to any one of Claims 1 to 3 or a pharmaceutically acceptable salt of the compound of formula (I).

7. Pharmaceutical composition, **characterized in that** it comprises a compound of formula (I) according to any one of Claims 1 to 3, a pharmaceutically acceptable salt of this compound and at least one pharmaceutically acceptable excipient.

8. Use of a compound of formula (I) according to any one of Claims 1 to 3 in the preparation of a medicament intended to treat and prevent pain, inflammatory diseases, autoimmune diseases, allergic diseases, infectious diseases, neurodegenerative diseases, cardiovascular diseases, cancers, gastrointestinal diseases, obesity, type II diabetes, insulin resistance and adipose tissue inflammation.
